# EUROPEAN PATENT SPECIFICATION

(11) **EP 3 765 601 B1**
(45) Date of publication and mention of the grant of the patent: **24.06.2026**
(21) Application number: 19714953.7
(22) Date of filing: 18.03.2019
(51) Int. Cl.: C12N 5/0781, C07K 14/47, A61K 35/17, A61P 21/00, C12N 15/64

(54) **B CELLS GENETICALLY ENGINEERED TO SECRETE FOLLISTATIN AND METHODS OF USING THE SAME TO TREAT FOLLISTATIN-RELATED DISEASES, CONDITIONS, DISORDERS AND TO ENHANCE MUSCLE GROWTH AND STRENGTH**
GENETISCH MANIPULIERTE B-ZELLEN ZUR SEZERNIERUNG VON FOLLISTATIN UND VERFAHREN ZUR VERWENDUNG DAVON ZUR BEHANDLUNG VON FOLLISTATINBEDINGTEN KRANKHEITEN, LEIDEN, STÖRUNGEN UND ZUR ERHÖHUNG DES MUSKELWACHSTUMS UND DER MUSKELKRAFT
LYMPHOCYTES B GÉNÉTIQUEMENT MODIFIÉES POUR SÉCRÉTER DE LA FOLLISTATINE ET LEURS MÉTHODES D'UTILISATION POUR TRAITER DES MALADIES, DES ÉTATS, DES TROUBLES LIÉS À LA FOLLISTATINE ET POUR AMÉLIORER LA CROISSANCE ET LA RÉSISTANCE MUSCULAIRES

(30) Priority: 16.03.2018 US 201862644362 P; 16.03.2018 US 201862644356 P
(43) Date of publication of application: 20.01.2021
(73) Proprietor: Immusoft Corporation, Seattle, WA 98103 (US)
(72) Inventor: SCHOLZ, Matthew Rein, Seattle, Washington 98103 (US); HERBIG, Eric J., Seattle, Washington 98103 (US); MCIVOR, R. Scott, Seattle, Washington 98103 (US)
(74) Representative: Schiweck Weinzierl Koch Patentanwälte Partnerschaft mbB
(86) International application number: PCT/US2019/022821
(87) International publication number: WO 2019/178613

(56) References cited:
- WO-A2-2016/057975
- US-A1- 2016 083 440
- US-A1- 2016 256 526
- US-B2- 7 887 793
- B. F. BENABDALLAH ET AL: "Overexpression of Follistatin in Human Myoblasts Increases Their Proliferation and Differentiation, and Improves the Graft Success in SCID Mice", CELL TRANSPLANTATION, vol. 18, no. 7, 1 July 2009 (2009-07-01), US, pages 709 - 718, XP055588847, ISSN: 0963-6897, DOI: 10.3727/096368909X470865
- CHUNXIA ZHAO ET AL: "Overcoming Insulin Insufficiency by Forced Follistatin Expression in [beta] -cells of db/db Mice", MOLECULAR THERAPY, vol. 23, no. 5, 1 May 2015 (2015-05-01), GB, pages 866 - 874, XP055525294, ISSN: 1525-0016, DOI: 10.1038/mt.2015.29
- JANAIAH KOTA ET AL: "Follistatin Gene Delivery Enhances Muscle Growth and Strength in Nonhuman Primates", SCIENCE TRANSLATIONAL MEDICINE, AMERICAN ASSOCIATION FOR THE ADVANCEMENT OF SCIENCE (A A A S), US, vol. 1, no. 6, 11 November 2009 (2009-11-11), pages 6ra15 - 1, XP002752988, ISSN: 1946-6242, DOI: 10.1126/SCITRANSLMED.3000112
- MENDELL J R ET AL: "A phase 1/2a follistatin gene therapy trial for becker muscular dystrophy", MOLECULAR THERAPY, NATURE PUBLISHING GROUP, GB, vol. 23, no. 1, 1 January 2015 (2015-01-01), pages 192 - 201, XP002752989, ISSN: 1525-0024, [retrieved on 20141017], DOI: 10.1038/MT.2014.200

## Description

### CROSS REFERENCE TO RELATED APPLICATIONS

This application claims priority to U.S. Provisional Application No. 62/644,362, filed on March 16, 2018, and U.S. Provisional Application No. 62/644,356, filed on March 16, 2018.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is IMCO-008_01WO_ST25.txt. The text file is 12 KB, was created on March 18, 2019, and is being submitted electronically via EFS-Web.

### BACKGROUND

### Technical Field

The present invention relates to recombinant B cell comprising a follistatin gene, an in vitro method of producing recombinant B cells and uses thereof. The present disclosure relates to the use of B cells for long term *in vivo* delivery of a therapeutic agent, such as follistatin, and in particular to administering single and multiple dosages of the B cells to a subject (e.g., a human).

### Description of the Related Art

Muscular dystrophies (MD) are progressive inherited neuromuscular disorders that are characterized by muscle wasting and weakness (Emery (2002) The Lancet, 359:687-695). Many forms of muscular dystrophies are fatal and currently incurable.

Duchenne muscular dystrophy (DMD) is the most common X-linked neuromuscular disease. The disease is caused by mutations in the DMD gene coding for dystrophin. Alteration or absence of this protein results in abnormal sarcolemmal membrane tearing. An abnormal variation in diameter of muscle fibers (atrophic and hypertrophic fibers) in proximal muscles and ongoing muscle damage are hallmarks of the disease. Damaged muscle releases the intracellular enzyme creatine kinase (CK). As a result, the serum CK levels in DMD patients are characteristically high (up to 10 times the normal). The pathophysiologic cascade is compounded by tissue inflammation, myofiber necrosis and replacement of muscle with fibrofatty tissue.

Another allelic variant of the DMD gene causes a milder form of MD known as Becker muscular dystrophy (BMD). BMD is clinically similar to DMD but the onset of symptoms occurs later in life.

Many pharmacological agents have been tried in MD but none has proved effective in arresting the course of the disease. The current modality of treatment is still in the realm of physical medicine and rehabilitation.

A number of trials using corticosteroids (e.g., prednisone and/or its derivatives) have demonstrated improvement in individuals with MD, particularly in the short-term. Although the exact mechanism by which corticosteroids alleviate the disease phenotype is unclear, corticosteroids are thought to act by reducing inflammation, suppressing the immune system, improving calcium homeostasis, upregulating expression of compensatory proteins, and increasing myoblast proliferation (Khurana et al. (2003) Nat. Rev. Drug Discovery 2:279-386). However, corticosteroids administered over time can induce muscle atrophy, which primarily affects proximal muscles-the very same muscles that are affected in DMD and BMD. The corticosteroid-induced muscle and other side effects may limit the long-term effectiveness of corticosteroid therapy.

The transforming growth factor-beta (TGF-beta) superfamily contains a variety of growth factors that share common sequence elements and structural motifs. These proteins are known to exert biological effects on a large variety of cell types in both vertebrates and invertebrates. Members of the superfamily perform important functions during embryonic development in pattern formation and tissue specification and can influence a variety of differentiation processes, including adipogenesis, myogenesis, chondrogenesis, cardiogenesis, hematopoiesis, neurogenesis, and epithelial cell differentiation. The family is divided into two general branches: the BMP/GDF and the TGF-beta/Activin/BMP10 branches, whose members have diverse, often complementary effects. By manipulating the activity of a member of the TGF-beta family, it is often possible to cause significant physiological changes in an organism. For example, the Piedmontese and Belgian Blue cattle breeds carry a loss-of-function mutation in the GDF8 (also called myostatin) gene that causes a marked increase in muscle mass. Grobet et al., Nat. Genet. 1997, 17(1):71-4. Furthermore, in humans, inactive alleles of GDF8 are associated with increased muscle mass and, reportedly, exceptional strength. Schuelke et al., N Engl J Med 2004, 350:2682-8. Moreover, mice genetically engineered to express either a dominant negative activin receptor IIB (ActRIIB) or to express follistatin have exceptional muscle mass (Lee, SJ and McPherron, AC, Proc Natl Acad Sci U S A. 2001 Jul 31;98(16):9306-11) and follistatin overexpression in non-human primates enhances muscle growth and strength. Kota J, et al., Sci Transl Med. 2009 Nov 11;1(6).

Thus, there is a need for methods of delivering agents that function as potent regulators of TGF-beta signaling.

Current methods for treating chronic diseases and disorders include direct infusion of a therapeutic agent (e.g., a therapeutic polypeptide), gene therapy via a viral vector, and adoptive transfer of stem cells (e.g., hematopoietic stem cell transfer). However, each of these methods have disadvantages. Injection of a recombinant therapeutic protein suffers from the finite half-life of the protein, and all three methods provide sub-optimal tissue penetration by the therapeutic agent. Altering endogenous tissues to produce a therapeutic agent, such as via injection of recombinant adeno-associated virus (AAV) and lentiviral vectors, generally results in the therapeutic agent being produced from a centralized location. Production of the therapeutic agent from one location increases the chances for localized toxicity in the producing tissues. Additionally, as recombinant viruses are viewed as foreign, it is unlikely viral vectors can be administered multiple times without causing an adverse reaction, meaning that there is a single injection opportunity to achieve the correct dosage of the therapeutic agent. Given the biological variation inherent in a procedure such as *in vivo* introduction of nucleic acids into cells using a virus, it would be very tenuous to achieve a desired dosage under the constraints of a single injection.

Accordingly, there still remains a need in the art for the long-term treatment for many chronic diseases and disorders related to TGF-beta signaling.

### SUMMARY OF THE EMBODIMENTS

The invention is set out in the appended set of claims. The aspects of the description, which do not fall within the scope of said claims are provided for illustrative purposes only and do not form part of the present invention. The present disclosure relates, generally, to compositions and methods for administering and dosing genetically modified B cell compositions for treating chronic diseases and disorders. In various embodiments, the present disclosure relates to compositions and methods for administering and dosing B cells genetically modified to express a polypeptide capable of modulating TGF-beta signaling (e.g., a follistatin polypeptide). In some particular embodiments, the present disclosure relates to compositions and methods for administering and dosing B cells genetically modified to express a follistatin polypeptide. In certain particular embodiments, the present disclosure relates to compositions and methods for administering and dosing B cells genetically modified to express a follistatin polypeptide having an amino acid sequence of SEQ ID NO: 1, SEQ ID NO: 2, SEQ ID NO: 3, or SEQ ID NO: 4. Such B cells may be used in various embodiments, e.g., to increase muscle size or strength in a subject (e.g., a human). The present disclosure provides these and other advantages as described in the detailed description.

In some embodiments, the present description relates to, a recombinant B cell comprising a follistatin gene. In some embodiments, the follistatin gene is operably linked to a promoter. In some embodiments, the follistatin gene is a human follistatin gene. In some embodiments, the follistatin gene is a human follistatin FST-344 splice site variant. In some embodiments, the B cell is a human B cell. In some embodiments, the B cell has been transduced with the follistatin gene. In some embodiments, the B cell comprises the follistatin gene because it has been transduced with the follistatin gene using a sleeping beauty transposon system. In some embodiments, the B cell expresses the follistatin gene due to transduction with a virus carrying the follistatin gene. In some embodiments, the B cell comprises the follistatin gene because it has been transduced with a retrovirus, lentivirus, adenovirus or adeno-associated virus comprising the follistatin gene. In some embodiments, the B cell is engineered to contain the follistatin gene using a targeted integration approach. In some embodiments, the targeted integration utilizes one or more zinc finger nucleases, transcription activator like effector nucleases (TALENs), and / or CRISPR/Cas systems including, but not limited to CRISPR/Cas9 systems. In some embodiments, the B cell is engineered to contain the follistatin gene by introducing a Follistatin-encoding nucleic acid using a method selected from the group consisting of retroviral vectors, lentiviral vectors, adeno-associated virus vectors, adenovirus vectors, any other RNA or DNA virus vectors, non-viral DNA and/or RNA encoding Follistatin introduced using chemical or physical means such and lipofection, polycation complexation, electroporation, and the like. In some embodiments, the follistatin gene is secreted by the recombinant B cell. In some aspects, the recombinant B cell is derived from a B cell obtained from the subject or a B cell derived from a cell obtained from the subject. In some aspects, the recombinant B cell is derived from a B cell progenitor obtained from the subject. In some aspects, the recombinant B cell is derived from a cell obtained from the subject that has been dedifferentiated into the B cell or a B cell progenitor. In some aspects, the recombinant B cell is engineered by
(a) collecting and isolating immune cells from the blood of the subject;
(b) transducing the cells with DNA encoding the follistatin;
(c) expanding selected cells *ex vivo;* and
(d) differentiating the expanded cells *ex vivo* into plasma cells and/or plasmablasts.

In some aspects, the isolated immune cells from step a are CD19 positive cells. In some aspects, the step b transducing is via electroporation. In some aspects, the electroporation utilized the sleeping beauty transposon system. In some aspects, the differentiated cells are CD38(+) and CD20(-). In some aspects, the present disclosure relates to a method comprising administering such a recombinant B cell to a subject.

In some aspects, the present invention relates to a method of delivering follistatin to a subject in need thereof comprising administering a recombinant B cell comprising a follistatin gene. In some embodiments, the present invention relates to method of delivering follistatin to a subject in need thereof comprising administering to the subject any one of the recombinant B cells disclosed herein that express a follistatin polypeptide. In some aspects, the subject is a mammal. In some aspects, the subject is a human. In some aspects, the subject has a muscle disorder. In some embodiments, the muscle disorder is a muscular dystrophies. In some embodiments, the muscular dystrophy is selected from Duchenne muscular dystrophy, Becker's muscular dystrophy and fascioscapulohumeral muscular dystrophy. In some embodiments, the muscle disorder is an inflammatory muscle disorders. In some embodiments, the inflammatory muscle disorder is inclusion body myositis. In some embodiments, the muscle disorder is a muscle injury or trauma. In some embodiments, the muscle disorder is muscle disuse. In some embodiments, the muscle disuse occurs after prolonged bed rest or limb immobilization. In some embodiments, the muscle disorder is a muscle atrophy or weakening. In some embodiments the muscle atrophy or weakening is caused by aging, cancer, or a chronic diseases. In some embodiments the muscle atrophy or weakening is due to sarcopenia. In some embodiments the muscle atrophy or weakening is due to spinal muscular atrophy (SMA). In some embodiments the muscle atrophy or weakening is due to amyotrophic lateral sclerosis (ALS). In some embodiments the muscle atrophy or weakening is due to Pompe disease. In some embodiments, the subject has muscle that is healthy. In some embodiments, administration of the B cell that expresses a follistatin polypeptide to the subject that has healthy muscle increases the subject's muscle size or strength.

In some particular embodiments, the present disclosure relates to a method for treating a muscular dystrophy comprising administering to a subject with muscular dystrophy a B cell expressing a follistatin polypeptide. In some embodiments, the subject has Becker Muscular Dystrophy. In some embodiments, the administering of the recombinant B cell to the subject effects treatment of a disease, disorder, or condition of the subject. In some embodiments, the administering of the recombinant B cell to the subject effects treatment of a muscular dystrophy. In some embodiments, the administering of the recombinant B cell to the subject causes the subject to gain weight. In some embodiments, the subject gains at least about 4% body weight. In some embodiments, significant gains in body weight occur within 30 days. In some embodiments, significant gains in body weight occur in about 30 days. In some embodiments, the administering of the recombinant B cell to the subject causes the subject to gain muscle In some embodiments, the administering of the recombinant B cell to the subject causes the subject to become stronger. In some embodiments, the administering of the recombinant B cell results in an increase in the subjects plasma levels of follistatin.

In some embodiments, the present invention relates to a method of treating, preventing, or ameliorating a muscular dystrophy by administering a recombinant B cell comprising a follistatin gene. In some embodiments, the method of treating, preventing, or ameliorating a muscular dystrophy comprises administering any one of the recombinant B cells disclosed herein. In some embodiments, the method comprises administering two or more sequential doses of genetically modified B cells to a subject. In some embodiments, the administering comprises two or more doses of the genetically modified B cells at sub-optimal single-dose concentrations. In some embodiments, administering comprises three or more doses of genetically modified B cells. In some embodiments, the genetically modified B cells are autologous to the subject. In some embodiments, the genetically modified B cells are allogeneic to the subject. In some embodiments, the subject is human. In some embodiments, the genetically modified B cells are CD20-, CD38-, and CD138-. In some embodiments, the genetically modified B cells are CD20-, CD38+, and CD138+. In some embodiments, the genetically modified B cells are CD20-, CD38+, and CD138-. In some embodiments, the administering comprises intravenous, intraperitoneal, subcutaneous, or intramuscular injection. In some embodiments, the administering comprises intravenous injection. In some embodiments, the genetically modified B cells are engineered on Day 2 or Day 3 after culturing. In some embodiments, the genetically modified B cells are engineered using a method comprising electroporation. In some embodiments, the genetically modified B cells are harvested for administration to a subject on Day 4, Day 5, Day 6, or Day 7 in culture after engineering. In some embodiments, the genetically modified B cells are harvested for administration to a subject on Day 8 or later in culture after engineering. In some embodiments, the genetically modified B cells are harvested for administration to a subject on Day 10 or earlier in culture after engineering. In some embodiments, the harvested genetically modified B cells do not produce significant levels of inflammatory cytokines. In some embodiments, the genetically modified B cells are harvested at a time-point in culture at which it is determined that they do not produce significant levels of inflammatory cytokines. In some embodiments, the genetically modified B cells are grown in a culture system that comprises each of IL-2, IL-4, IL-10, IL-15, IL-31, and a multimerized CD40 ligand throughout the entire culture period pre- and post-engineering. In some embodiments, the multimerized CD40 ligand is a HIS tagged CD40 ligand that is multimerized using an anti-his antibody. In some embodiments, the method further comprises expanding the genetically modified B cells prior to the administering to the subject. In some embodiments, the final population of expanded genetically modified B cells demonstrates a high degree of polyclonality. In some embodiments, any particular B cell clone in the final population of expanded genetically modified B cells comprises less than 0.2% of the total B cell population. In some embodiments, any particular B cell clone in the final population of expanded genetically modified B cells comprises less than 0.05% of the total B cell population. In some embodiments, the genetically modified B cells comprise a polynucleotide encoding a human DHFR gene with enhanced resistance to methotrexate. In some embodiments, the human DHFR gene with enhanced resistance to methotrexate contains substitution mutations of leucine to tyrosine at amino acid 22 and phenylalanine to serine at amino acid 31. In some embodiments, the method comprises treating the genetically modified B cells with methotrexate prior to harvesting for administration. In some embodiments, the methotrexate treatment is between 100 nM and 300 nM. In some embodiments, the methotrexate treatment is 200 nM. In some embodiments, the genetically modified B cells migrate to diverse tissues upon administration to the subject. In some embodiments of the method, at least one genetically modified B cell out of the population of genetically modified B cells that are administered to the subject migrates to one or more tissue selected from the group consisting of bone marrow, intestine, muscle, spleen, kidney, heart, liver, lung and brain. In some embodiments of the method, at least one genetically modified B cell out of the population of genetically modified B cells that are administered to the subject migrates to the subject's bone marrow, intestine, muscle, spleen, kidney, heart, liver, lung and brain.

In some embodiments, the present invention provides a modified B cell transduced to express both a follistatin gene and a dihydrofolate reductase (DHFR) gene.

### BRIEF DESCRIPTION OF THE DRAWINGS

Figure 1 shows that treatment with follistatin-expressing B cells results in increased follistatin levels in mouse plasma. From left to right in each of control and treated groups: bars correspond to Day 21, 28, and 35, respectively.
Figure 2 shows that plasma levels of follistatin in mice treated with follistatin-expressing B cells correlate with levels of human IgG, which is a surrogate marker for engraftment. FIGS. 2A-2D show plasma levels of follistatin in four separate mice treated with B cells expressing follistatin.
Figure 3 shows percent change in weight in mice treated or not treated with follistatin-expressing B cells.
Figure 4 shows strength assessments in mice treated or not treated with follistatin-expressing B cells. FIG. 4A shows strength as assessed by the Front Leg Grip test. FIG. 4B shows strength as assessed by the Four Leg Grip test. FIG. 4C shows strength as assessed by the Hanging Test. Percent improvements provided below the graphs show the average percent improvement in the treated group as compared to the untreated group.
Figure 5 shows *in vitro* follistatin expression in follistatin-expressing B cells. FIG. 5A shows follistatin protein expression as determined by ELISA. FIG. 5B shows follistatin mRNA expression as determined by RT-PCR.

### DETAILED DESCRIPTION

The invention is set out in the appended set of claims. The aspects of the description, which do not fall within the scope of said claims are provided for illustrative purposes only and do not form part of the present invention. The practice of the present invention will employ, unless indicated specifically to the contrary, conventional methods of molecular biology, recombinant DNA techniques, protein expression, and protein / peptide / carbohydrate chemistry within the skill of the art, many of which are described below for the purpose of illustration. Such techniques are explained fully in the literature. See, e.g., Sambrook, et al., Molecular Cloning: A Laboratory Manual (3rd Edition, 2000); DNA Cloning: A Practical Approach, vol. I & II (D. Glover, ed.); Oligonucleotide Synthesis (N. Gait, ed., 1984); Oligonucleotide Synthesis: Methods and Applications (P. Herdewijn, ed., 2004); Nucleic Acid Hybridization (B. Hames & S. Higgins, eds., 1985); Nucleic Acid Hybridization: Modern Applications (Buzdin and Lukyanov, eds., 2009); Transcription and Translation (B. Hames & S. Higgins, eds., 1984); Animal Cell Culture (R. Freshney, ed., 1986); Freshney, R.I. (2005) Culture of Animal Cells, a Manual of Basic Technique, 5th Ed. Hoboken NJ, John Wiley & Sons; B. Perbal, A Practical Guide to Molecular Cloning (3rd Edition 2010); Farrell, R., RNA Methodologies: A Laboratory Guide for Isolation and Characterization (3rd Edition 2005). The publications discussed above are provided solely for their disclosure before the filing date of the present application. Nothing herein is to be construed as an admission that the invention is not entitled to antedate such disclosure by virtue of prior invention.

### DEFINITIONS AND ABBREVIATIONS

Unless defined otherwise, all technical and scientific terms used herein have the same meaning as commonly understood by one of ordinary skill in the art to which this invention belongs. As used in the specification and appended claims, unless specified to the contrary, the following terms have the meaning indicated. With regard to this specification, any time a definition of a term as defined herein, differs from a definition given for that same term in an incorporated reference, the definition explicitly defined herein is the correct definition of the term.

The words "a" and "an" denote one or more, unless specifically noted.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length. In any embodiment discussed in the context of a numerical value used in conjunction with the term "about," it is specifically contemplated that the term about can be omitted.

A "composition" can comprise an active agent and a carrier, inert or active, e.g., a pharmaceutically acceptable carrier, diluent or excipient. In particular embodiments, the compositions are sterile, substantially free of endotoxins or non-toxic to recipients at the dosage or concentration employed.

Unless the context requires otherwise, throughout the present specification and claims, the word "comprise" and variations thereof, such as, "comprises" and "comprising" are to be construed in an open and inclusive sense, that is, as "including, but not limited to".

By "consisting of" is meant including, and limited to, whatever follows the phrase "consisting of." Thus, the phrase "consisting of" indicates that the listed elements are required or mandatory and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

Reference throughout this specification to "biological activity" or "bioactivity" refers to any response induced in an in vitro assay or in a cell, tissue, organ, or organism, (e.g., an animal, or a mammal, or a human) as the result of administering any compound, agent, polypeptide, conjugate, pharmaceutical composition contemplated herein. Biological activity may refer to agonistic actions or antagonistic actions. The biological activity may be a beneficial effect; or the biological activity may not be beneficial, i.e. a toxicity. In some embodiments, biological activity will refer to the positive or negative effects that a drug or pharmaceutical composition has on a living subject, e.g., a mammal such as a human. Accordingly, the term "biologically active" is meant to describe any compound possessing biological activity, as herein described. Biological activity may be assessed by any appropriate means currently known to the skilled artisan. Such assays may be qualitative or quantitative. The skilled artisan will readily appreciate the need to employ different assays to assess the activity of different polypeptides; a task that is routine for the average researcher. Such assays are often easily implemented in a laboratory setting with little optimization requirements, and more often than not, commercial kits are available that provide simple, reliable, and reproducible readouts of biological activity for a wide range of polypeptides using various technologies common to most labs. When no such kits are available, ordinarily skilled researchers can easily design and optimize in-house bioactivity assays for target polypeptides without undue experimentation; as this is a routine aspect of the scientific process.

Reference to the term "e.g." is intended to mean "e.g., but not limited to" and thus it should be understood that whatever follows is merely an example of a particular embodiment, but should in no way be construed as being a limiting example. Unless otherwise indicated, use of "e.g." is intended to explicitly indicate that other embodiments have been contemplated and are encompassed by the present invention.

Reference throughout this specification to "embodiment" or "one embodiment" or "an embodiment" or "some embodiments" or "certain embodiments" means that a particular feature, structure or characteristic described in connection with the embodiment is included in at least one embodiment of the present invention. Thus, the appearances of the phrases "in one embodiment" or "in an embodiment" or "in certain embodiments" in various places throughout this specification are not necessarily all referring to the same embodiment. Furthermore, the particular features, structures, or characteristics may be combined in any suitable manner in one or more embodiments.

An "increased" or "enhanced" amount is typically a "statistically significant" amount, and may include an increase that is 1.1, 1.2, 1.3, 1.4, 1.5, 1.6, 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 2.1, 2.2, 2.3, 2.4, etc.) an amount or level described herein. Similarly, a "decreased" or "reduced" or "lesser" amount is typically a "statistically significant" amount, and may include a decrease that is about 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (e.g., 100, 500, 1000 times) (including all integers and decimal points in between and above 1, e.g., 1.5, 1.6, 1.7. 1.8, etc.) an amount or level described herein.

The terms "in vitro", "ex vivo", and "in vivo" are intended herein to have their normal scientific meanings. Accordingly, e.g., "in vitro" is meant to refer to experiments or reactions that occur with isolated cellular components, such as, e.g., an enzymatic reaction performed in a test tube using an appropriate substrate, enzyme, donor, and optionally buffers / cofactors. "Ex vivo" is meant to refer to experiments or reactions carried out using functional organs or cells that have been removed from or propagated independently of an organism. "In vivo" is meant to refer to experiments or reactions that occur within a living organism in its normal intact state.

"Mammal" includes humans and both domestic animals such as laboratory animals and household pets, (e.g., cats, dogs, swine, cattle, sheep, goats, horses, and rabbits), and non-domestic animals such as wildlife and the like.

"Optional" or "optionally" means that the subsequently described event, or circumstances, may or may not occur, and that the description includes instances where said event or circumstance occurs and instances in which it does not.

"Pharmaceutical composition" refers to a formulation of a compound (e.g. a therapeutically useful polypeptide) and a medium generally accepted in the art for the delivery of the compound to an animal, e.g., humans. Such a medium may include any pharmaceutically acceptable carriers, diluents or excipients therefore.

"Pharmaceutically effective excipients" and "pharmaceutically effective carriers" are well known to those of skill in the art, and methods for their preparation are also readily apparent to the skilled artisan. Such compositions, and methods for their preparation, may be found, e.g., in Remington's Pharmaceutical Sciences, 19th Edition (Mack Publishing Company, 1995).

The terms "polynucleotide", "nucleotide", "nucleotide sequence", and "nucleic acid" are used interchangeably. They refer to a polymeric form of nucleotides of any length, either deoxyribonucleotides or ribonucleotides, or analogs thereof. Polynucleotides may have any three dimensional structure, and may perform any function known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may include non-nucleotide components. A polynucleotide may be further modified after polymerization, such as by conjugation with a labeling component.

A "subject," as used herein, includes any animal that exhibits a disease or symptom, or is at risk for exhibiting a disease or symptom, which can be treated with an agent of the invention. Suitable subjects include laboratory animals (such as mouse, rat, rabbit, or guinea pig), farm animals, and domestic animals or pets (such as a cat or dog). Non-human primates and, preferably, human patients, are included.

"Substantially" or "essentially" means of ample or considerable amount, quantity, size; nearly totally or completely; for instance, 95% or greater of some given quantity.

"Therapeutic agent" refers to any compound that, when administered to a subject, (e.g., preferably a mammal, more preferably a human), in a therapeutically effective amount is capable of effecting treatment of a disease or condition as defined below.

"Therapeutically effective amount" or "Therapeutically effective dose" refers to an amount of a compound of the invention that, when administered to a subject, (e.g., preferably a mammal, more preferably a human), is sufficient to effect treatment, as defined below, of a disease or condition in the animal. The amount of a compound of the invention that constitutes a "therapeutically effective amount" will vary depending on the compound, the condition and its severity, the manner of administration, and the age of the animal to be treated, but can be determined routinely by one of ordinary skill in the art having regard to his own knowledge and to this disclosure.

"Treating" or "treatment" as used herein covers the treatment of the disease or condition of interest in a subject, preferably a human, having the disease or condition of interest, and includes: (i) preventing or inhibiting the disease or condition from occurring in a subject, in particular, when such subject is predisposed to the condition but has not yet been diagnosed as having it; (ii) inhibiting the disease or condition, *i.e.,* arresting its development; (iii) relieving the disease or condition, *i.e.,* causing regression of the disease or condition; or (iv) relieving the symptoms resulting from the disease or condition. As used herein, the terms "disease," "disorder," and "condition" may be used interchangeably or may be different in that the particular malady, injury or condition may not have a known causative agent (so that etiology has not yet been worked out), and it is, therefore, not yet recognized as an injury or disease but only as an undesirable condition or syndrome, wherein a more or less specific set of symptoms have been identified by clinicians.

### OVERVIEW

The present invention relates to, *inter alia,* autologous and/or allogeneic B cells that have been altered through introduction of nucleic acids to produce follistatin and also relates to methods of administering the modified B cells (e.g., to treat a disease, disorder, or condition, e.g., a muscle disorder such as muscular dystrophy). In some embodiments, the terms "engineered B cell", "genetically engineered B cell", "modified B cell" and "genetically modified B cell" are used interchangeably herein to refer to such altered B cells that comprises one or more nucleic acids (e.g., a transgene) to produce follistatin (e.g., a transgene that enables expression of a follistatin polypeptide such as a therapeutic follistatin polypeptide). Specifically, the modified B cells can be administered as a single dosage or multiple dosages.

Accordingly, the methods for administering modified B cell compositions described herein are useful for long term *in vivo* delivery and expression of follistatin. The present disclosure relates generally to methods for achieving sufficient enrichment and number of cells producing follistatin and sufficient levels of follistatin *in vivo* while ensuring product safety.

As used herein, the phrases "long term *in vivo* survival" and "long term survival" refer to the survival of the modified B cells described herein for 10 or more days post administration in a subject. Long term survival may be measured in days, weeks, or even years. In one embodiment, a majority of the modified B cells survive *in vivo* for 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more days post-administration. In one embodiment, a majority of the modified B cells survive *in vivo* for 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 or more weeks post-administration. In another embodiment, the modified B cells survive *in vivo* for 1, 1.5, 2, 2.5, 3, 3.5, 4, 4.5, 5, 6, 7, 8, 9, 10, 11, 12, 13, 14, 15, 16, 17, 18, 19, 20, 25, 30 or more years. Additionally, while the modified B cells described herein may survive *in vivo* for 10 or more days, it is understood that a majority of the modified B cells survive *in vivo* for 1, 2, 3, 4, 5, 6, 7, 8, 9 or more days post-administration. Accordingly, it is contemplated that modified B cells described herein are useful for short-term treatment (e.g., 4 days) and long-term treatment (e.g., 30 or more days) methods.

### B cells

After leaving the bone marrow, a B cell acts as an antigen presenting cell (APC) and internalizes antigens. Antigen is taken up by the B cell through receptor-mediated endocytosis and processed. Antigen is processed into antigenic peptides, loaded onto MHC II molecules, and presented on the B cell extracellular surface to CD4+ T helper cells. These T cells bind to the MHC ll/antigen molecule and cause activation of the B cell. Upon stimulation by a T cell, the activated B cell begins to differentiate into more specialized cells. Germinal center B cells may differentiate into long-lived memory B cells or plasma cells. Further, secondary immune stimulation may result in the memory B cells giving rise to additional plasma cells. The formation of plasma cells from either memory or non-memory B cells is preceded by the formation of precursor plasmablasts that eventually differentiate into plasma cells, which produce large volumes of antibodies (see *e.g.,* Trends Immunol. 2009 June; 30(6): 277-285; Nature Reviews, 2005, 5:231 -242). Plasmablasts secrete more antibodies than B cells, but less than plasma cells. They divide rapidly, and they continue to internalize antigens and present antigens to T cells. Plasmablasts have the capacity to migrate to sites of chemokine production (*e.g*. in bone marrow) whereby they may differentiate into long-lived plasma cells. Ultimately, a plasmablast may either remain as a plasmablast for several days and then die or irrevocably differentiate into a mature, fully differentiated plasma cell. Specifically, plasmablasts that are able home to tissues containing plasma cell survival niches (*e.g.,* in bone marrow) are able to displace resident plasma cells in order to become long lived plasma cells, which may continue to secrete high levels of proteins for years.

The B cells used in the methods described herein (e.g., to express follistatin) include pan B cells, memory B cells, plasmablasts, and/or plasma cells. In one embodiment, the modified B cells are memory B cells (e.g., that are modified to express follistatin). In one embodiment, the modified B cells are plasmablasts (e.g., that are modified to express follistatin). In one embodiment, the modified B cells are plasma cells (e.g., that are modified to express follistatin).

Terminally differentiated plasma cells typically do not express common pan-B cell markers, such as CD19 and CD20, and express relatively few surface antigens. Plasma cells express CD38, CD78, CD138 and interleukin-6 receptor (IL-6R) and lack expression of CD45, and these markers can be used, *e.g.,* by flow cytometry, to identify plasma cells. CD27 is also a good marker for plasma cells as naive B cells are CD27-, memory B cells are CD27+ and plasma cells are CD27++. Memory B cell subsets may also express surface IgG, IgM and IgD, whereas plasma cells do not express these markers on the cell surface. CD38 and CD138 are expressed at high levels on plasma cells (See Wikipedia, The Free Encyclopedia., "Plasma cell" Page Version ID: 404969441 ; Date of last revision: 30 December 2010 09:54 UTC, retrieved January 4, 2011 ; See also: Jourdan et al. Blood. 2009 Dec 10;114(25):5173-81; Trends Immunol. 2009 June; 30(6): 277-285; Nature Reviews, 2005, 5:231 - 242; Nature Med. 2010, 16:123-129; Neuberger, M. S.; Honjo, T.; Alt, Frederick W. (2004). Molecular biology of B cells. Amsterdam: Elsevier, pp. 189-191 ; Bertil Glader; Greer, John G.; John Foerster; Rodgers, George G.; Paraskevas, Frixos (2008). Wintrobe's Clinical Hematology, 2-Vol. Set. Hagerstwon, MD: Lippincott Williams & Wilkins. pp. 347; Walport, Mark; Murphy, Kenneth; Janeway, Charles; Travers, Paul J. (2008). Janeway's immunobiology. New York: Garland Science, pp. 387-388; Rawstron AC (May 2006). "Immunophenotyping of plasma cells". Curr Protoc Cytom).

"Quiescent", as used herein, refers to a cell state wherein the cell is not actively proliferating.

"Activated", as used herein, refers to a cell state wherein the cell is actively proliferating and/or producing cytokines in response to a stimulus.

The terms "differentiate" and "differentiated", as used herein, refer to changes in the phenotype of a cell from one cell type or state to another cell type or state. For example, a memory B cell that transitions to a plasma cell is differentiated.

The term "subject" is intended to include living organisms in which an adaptive immune response can be elicited (*e.g*., mammals). Examples of subjects include humans, dogs, cats, mice, rats, and non-human transgenic species thereof. In one embodiment, the subject is human. B cells can be obtained from a number of sources, including peripheral blood mononuclear cells (PBMCs), bone marrow, lymph node tissue, cord blood, tissue from a site of infection, spleen tissue, and tumors. In a preferred embodiment, the source of B cells is PBMCs. In certain embodiments of the present disclosure, any number of B cell lines available in the art, may be used.

In certain embodiments of the methods described herein, B cells can be obtained from a unit of blood collected from a subject using any number of techniques known to the skilled artisan, such as FICOLL^{™} (copolymers of sucrose and epichlorohydrin that may be used to prepare high density solutions) separation. In one preferred embodiment, cells from the circulating blood of an individual are obtained by apheresis or leukapheresis. The apheresis product typically contains lymphocytes, including T cells, monocytes, granulocytes, B cells, other nucleated white blood cells, red blood cells, and platelets. In one embodiment, the cells collected by apheresis may be washed to remove the plasma fraction and to place the cells in an appropriate buffer or media for subsequent processing steps. In one embodiment of the methods described herein, the cells are washed with phosphate buffered saline (PBS). In an alternative embodiment, the wash solution lacks calcium and may lack magnesium or may lack many if not all divalent cations. As those of ordinary skill in the art would readily appreciate a washing step may be accomplished by methods known to those in the art, such as by using a semi-automated "flow-through" centrifuge (for example, the Cobe 2991 cell processor) according to the manufacturer's instructions. After washing, the cells may be resuspended in a variety of biocompatible buffers, such as, for example, PBS. Alternatively, the undesirable components of the apheresis sample may be removed and the cells directly resuspended in culture media.

B cells may be isolated from peripheral blood or leukapheresis using techniques known in the art. For example, PBMCs may be isolated using FICOLL^{™} (Sigma-Aldrich, St Louis, MO) and CD19+ B cells purified by negative or positive selection using any of a variety of antibodies known in the art, such as the Rosette tetrameric complex system (StemCell Technologies, Vancouver, Canada) or MACS^{™} MicroBead Technology (Miltenyi Biotec, San Diego, CA). In certain embodiments, memory B cells are isolated as described by Jourdan et al., (Blood. 2009 Dec 10; 114(25):5173-81). For example, after removal of CD2+ cells using anti-CD2 magnetic beads, CD19+ CD27+ memory B cells can be sorted by FACS. Bone marrow plasma cells (BMPCs) can be purified using anti-CD138 magnetic microbeads sorting or other similar methods and reagents. Human B cells may be isolated, e.g., using CD19 MicroBeads, human (Miltenyi Biotec, San Diego, CA). Human Memory B cell may be isolated, e.g., using the Memory B Cell Isolation Kit, human (Miltenyi Biotec, San Diego, CA).

Other isolation kits are commercially available, such as R&D Systems' MagCellect Human B Cell Isolation Kit (Minneapolis, MN). In certain embodiments, resting B cells may be prepared by sedimentation on discontinuous Percoll gradients, as described in (Defranco et al., (1982) J. Exp. Med. 155:1523).

In one embodiment, PBMCs are obtained from a blood sample using a gradient based purification (*e.g*., FICOLL^{™}). In another embodiment, PBMCs are obtained from apheresis based collection. In one embodiment, B cells are isolated from PBMCs by isolating pan B cells. The isolating step may utilize positive and/or negative selection. In one embodiment, the negative selection comprises depleting T cells using anti-CD3 conjugated microbeads, thereby providing a T cell depleted fraction. In a further embodiment, memory B cells are isolated from the pan B cells or the T cell depleted fraction by positive selection for CD27.

In one particular embodiment, memory B cells are isolated by depletion of unwanted cells and subsequent positive selection with CD27 MicroBeads. Unwanted cells, for example, T cells, NK cells, monocytes, dendritic cells, granulocytes, platelets, and erythroid cells may be depleted using a cocktail of biotinylated antibodies against CD2, CD14, CD16, CD36, CD43, and CD235a (glycophorin A), and Anti-Biotin MicroBeads.

In one embodiment, switched memory B cells are obtained. "Switched memory B cell" or "switched B cell," as used herein, refers to a B cell that has undergone isotype class switching. In one embodiment, switched memory B cells are positively selected for IgG. In another embodiment, switched memory B cells are obtained by depleting IgD and IgM expressing cells. Switched memory B cells may be isolated, e.g., using the Switched Memory B Cell Kit, human (Miltenyi Biotec, San Diego, CA).

For example, in one particular embodiment, non-target cells may be labeled with a cocktail of biotinylated CD2, CD14, CD16, CD36, CD43, CD235a (glycophorin A), Anti-IgM, and Anti-IgD antibodies. These cells may be subsequently magnetically labeled with Anti-Biotin MicroBeads. Highly pure switched memory B cells may be obtained by depletion of the magnetically labeled cells.

In a further embodiment the promoter sequence from a gene unique to memory B cells, such as, e.g., the CD27 gene (or other gene specific to memory B cells and not expressed in naive B cells) is used to drive expression of a selectable marker such as, e.g., mutated dihydrofolate reductase allowing for positive selection of the memory B cells in the presence of methotrexate. In another embodiment, the promoter sequence from a pan B cell gene such as, e.g., the CD19 gene is used to drive expression of a selectable marker such as, e.g., mutated dihydrofolate reductase allowing for positive selection of the memory B cells in the presence of methotrexate. In another embodiment T cells are depleted using CD3 or by addition of cyclosporin. In another embodiment, CD138+ cells are isolated from the pan B cells by positive selection. In yet another embodiment, CD138+ cells are isolated from PBMCs by positive selection. In another embodiment, CD38+ cells are isolated from the pan B cells by positive selection. In yet another embodiment, CD38+ cells are isolated from PBMCs by positive selection. In one embodiment, CD27+ cells are isolated from PBMCs by positive selection. In another embodiment, memory B cells and/or plasma cells are selectively expanded from PBMCs using *in vitro* culture methods available in the art.

### Culturing B Cells In Vitro

B cells, such as memory B cells, can be cultured using *in vitro* methods to activate and differentiate the B cells into plasma cells or plasmablasts or both. As would be recognized by the skilled person, plasma cells may be identified by cell surface protein expression patterns using standard flow cytometry methods. For example, terminally differentiated plasma cells express relatively few surface antigens, and do not express common pan-B cell markers, such as CD19 and CD20. Instead, plasma cells may be identified by expression of CD38, CD78, CD138, and IL-6R and lack of expression of CD45. CD27 may also be used to identify plasma cells as naive B cells are CD27-, memory B cells are CD27+ and plasma cells are CD27++. Plasma cells express high levels of CD38 and CD138.

In one embodiment, the B cells are CD138- memory B cells. In one embodiment, the B cells are CD138+ plasma cells. In one embodiment, the B cells are activated and have a cell surface phenotype of CD138-, CD27+.

In one embodiment, the B cells are CD20-, CD138- memory B cells. In one embodiment, the B cells are CD20-, CD138+ plasma cells. In one embodiment, the B cells are activated and have a cell surface phenotype of CD20-, CD138-, CD27+.

In one embodiment, the B cells are CD20-, CD38-, CD138- memory B cells. In one embodiment, the B cells are CD20-, CD38+, CD138+ plasma cells. In one embodiment, the B cells are activated and have a cell surface phenotype of CD20-CD38- CD138- CD27+.

In one embodiment, the B cells are contacted with one or more B cell activating factors, e.g., any of a variety of cytokines, growth factors or cell lines known to activate and/or differentiate B cells (see *e.g.,* Fluckiger, et al. Blood 1998 92: 4509-4520; Luo, et al., Blood 2009 1 13: 1422-1431 ). Such factors may be selected from the group consisting of, but not limited to, IL-1, IL-2, IL-3, IL-4, IL-5, IL-6, IL-7, IL-8, IL-9, IL-10, IL-1 1 , IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, and IL-35, IFN-γ, IFN-α, IFN-β, IFN-δ, C type chemokines XCL1 and XCL2, C-C type chemokines (to date including CCL1 -CCL28) and CXC type chemokines (to date including CXCL1 -CXCL17), and members of the TNF superfamily (*e.g*., TNF-α, 4-1 BB ligand, B cell activating factor (BLyS), FAS ligand, sCD40L (including multimeric versions of sCD40L; *e.g*., histidine-tagged soluble recombinant CD40L in combination with anti-poly-histidine mAb to group multiple sCD40L molecules together), Lymphotoxin, OX40L, RANKL, TRAIL), CpG, and other toll like receptor agonists (*e.g.,* CpG).

B cell activating factors may be added to *in vitro* cell cultures at various concentrations to achieve the desired outcome (*e.g*., expansion or differentiation). In one embodiment, a B cell activating factor is utilized in expanding the B cells in culture. In one embodiment, a B cell activating factor is utilized in differentiating the B cells in culture. In another embodiment, the B cell activating factor is utilized in both expanding and differentiating the B cells in culture. In one embodiment, the B cell activating factor is provided at the same concentration for expanding and differentiating. In another embodiment, the B cell activating factor is provided at a first concentration for expanding and at a second concentration for differentiating. It is contemplated that a B cell activating factor may be 1) utilized in expanding the B cells and not in differentiating the B cells, 2) utilized in differentiating the B cells and not in expanding the B cells, or 3) utilized in expanding and differentiating the B cells.

For example, in some embodiments, B cells are cultured with a B cell culture medium containing one or more B cell activating factors selected from CD40L, IL-2, IL-4, and IL-10 for expansion of the B cells. In one embodiment, the B cells are cultured with 0.25-5.0 µg/ml CD40L. In one embodiment, the concentration of CD40L is 0.5 µg/ml. In one embodiment a crosslinking agent (such as an anti-HIS antibody in combination with HIS-tagged CD40L) is used to create multimers of CD40L. In one embodiment molecules of CD40L are covalently linked or are held together using protein multimerization domains (e.g., the Fc region of an IgG or a leucine zipper domain). In one embodiment CD40L is conjugated to beads. In one embodiment CD40L is expressed from feeder cells. In one embodiment, the B cells are cultured with 1-10 ng/ml IL-2. In one embodiment, the concentration of IL-2 is 5 ng/ml. In one embodiment, the B cells are cultured with 1-10 ng/ml IL-4. In one embodiment, the concentration of IL-4 is 2 ng/ml. In one embodiment, the B cells are cultured with 10-100 ng/ml IL-10. In one embodiment, the concentration of IL-10 is 40 ng/ml.

In one embodiment, B cells are cultured with a B cell culture medium containing one or more B cell activating factors selected from CD40L, IL-2, IL-4, IL-10, IL-15 and IL-21 for expansion of the B cells. In one embodiment, the B cells are cultured with 0.25-5.0 µg/ml CD40L. In one embodiment, the concentration of CD40L is 0.5 µg/ml. In one embodiment a crosslinking agent (such as an anti-HIS antibody in combination with HIS-tagged CD40L) is used to create multimers of CD40L. In one embodiment molecules of CD40L are covalently linked or are held together using protein multimerization domains (e.g., the Fc region of an IgG or a leucine zipper domain). In one embodiment CD40L is conjugated to beads. In one embodiment CD40L is expressed from feeder cells. In one embodiment, the B cells are cultured with 1-10 ng/ml IL-2. In one embodiment, the concentration of IL-2 is 5 ng/ml. In one embodiment, the B cells are cultured with 1-10 ng/ml IL-4. In one embodiment, the concentration of IL-4 is 2 ng/ml. In one embodiment, the B cells are cultured with 10-100 ng/ml IL-10. In one embodiment, the concentration of IL-10 is 40 ng/ml. In one embodiment, the B cells are cultured with 50-150 ng/ml IL-15. In one embodiment, the concentration of IL-15 is 100 ng/ml. In one embodiment, the B cells are cultured with 50-150 ng/ml IL-21. In one embodiment, the concentration of IL-21 is 100 ng/ml. In a particular embodiment, the B cells are cultured with a B cell culture medium containing CD40L, IL-2, IL-4, IL-10, IL-15 and IL-21 for expansion of the B cells.

For example, in one embodiment, B cells are cultured with a B cell culture medium containing the B cell activating factors CD40L, IL-2, IL-4, IL-10, IL-15 and IL-21 for expansion of the B cells, wherein the CD40L is crosslinked with a crosslinking agent to create multimers of CD40L. Such a culture system may be maintained throughout an entire culture period (e.g., a 7 day culture period), in which the B cells are transfected or otherwise engineered to express a transgene of interest (e.g., an exogenous polypeptide such as, e.g., FST). The transgene may be integrated into the B cell (e.g., via a viral or non-viral vector). The transgene may be expressed in the B cell via use of a transposon. The transgene may be expressed in the B cell due to the targeted integration of the transgene into the B cell's genome. The targeted integration may be via homologous recombination. The homologous recombination may occur at a double strand break induced by a nuclease. The nuclease may be, e.g., a zinc finger nuclease, a TALE-nuclease (TALEN), a meganuclease (e.g., a homing endonuclease), or via a CRISPR / CAS9-nulease system.

In another example, in one embodiment, B cells are cultured with a B cell culture medium containing one or more B cell activating factors selected from CD40L, IFN-α, IL-2, IL-6, IL-10, IL-15, IL-21, and P-class CpG oligodeoxynucleotides (p-ODN) for differentiation of the B cells. In one embodiment, the B cells are cultured with 25-75 ng/ml CD40L. In one embodiment, the concentration of CD40L is 50 ng/ml. In one embodiment, the B cells are cultured with 250-750 U/ml IFN-α. In one embodiment the concentration of the IFN-α is 500 U/ml. In one embodiment, the B cells are cultured with 5-50 U/ml IL-2. In one embodiment the concentration of IL-2 is 20 U/ml. In one embodiment, the B cells are cultured with 25-75 ng/ml IL-6. In one embodiment, the concentration of IL-6 is 50 ng/ml. In one embodiment, the B cells are cultured with 10-100 ng/ml IL-10. In one embodiment, the concentration of IL-10 is 50 ng/ml. In one embodiment, the B cells are cultured with 1-20 ng/ml IL-15. In one embodiment, the concentration of IL-15 is 10 ng/ml. In one embodiment, the B cells are cultured with 10-100 ng/ml IL-21. In one embodiment, the concentration of IL-21 is 50 ng/ml. In one embodiment, the B cells are cultured with 1-50 µg/ml p-ODN. In one embodiment, the concentration of p-ODN is 10 µg/ml.

In one embodiment, B cells are contacted or cultured on feeder cells. In one embodiment, the feeder cells are a stromal cell line, *e.g*., murine stromal cell line S17 or MS5. In another embodiment, isolated CD19+ cells are cultured with one or more B cell activating factor cytokines, such as IL-10 and IL-4, in the presence of fibroblasts expressing CD40-ligand (CD40L, CD154). In one embodiment, CD40L is provided bound to a surface such as tissue culture plate or a bead. In another embodiment, purified B cells are cultured, in the presence or absence of feeder cells, with CD40L and one or more cytokines or factors selected from IL-10, IL-4, IL-7, p-ODN, CpG DNA, IL-2, IL-15, IL6, and IFN-α.

In another embodiment, B cell activating factors are provided by transfection into the B cell or other feeder cell. In this context, one or more factors that promote differentiation of the B cell into an antibody secreting cell and/or one or more factors that promote the longevity of the antibody producing cell may be used. Such factors include, for example, Blimp-1, TRF4, anti-apoptotic factors like Bcl-xl or Bcl5, or constitutively active mutants of the CD40 receptor. Further, factors which promote the expression of downstream signaling molecules such as TNF receptor-associated factors (TRAPs) may also be used in the activation/differentiation of the B cells. In this regard, cell activation, cell survival, and antiapoptotic functions of the TNF receptor superfamily are mostly mediated by TRAF1-6 (see *e.g.,* R.H. Arch, et al., Genes Dev. 12 (1998), pp. 2821-2830). Downstream effectors of TRAF signaling include transcription factors in the NF- κB and AP-1 family which can turn on genes involved in various aspects of cellular and immune functions. Further, the activation of NF-κB and AP-1 has been shown to provide cells protection from apoptosis via the transcription of antiapoptotic genes.

In another embodiment, Epstein Barr virus (EBV)-derived proteins are used for the activation and/or differentiation of B cells or to promote the longevity of the antibody producing cell. EBV-derived proteins include but are not limited to, EBNA-1, EBNA-2, EBNA-3, LMP-1, LMP-2, EBER, miRNAs, EBV-EA, EBV-MA, EBV-VCA and EBV-AN.

In certain embodiments, contacting the B cells with B cell activation factors using the methods provided herein leads to, among other things, cell proliferation (*i.e*., expansion), modulation of the IgM+ cell surface phenotype to one consistent with an activated mature B cell, secretion of Ig, and isotype switching. CD19+ B cells may be isolated using known and commercially available cell separation kits, such as the MiniMACS^{™} cell separation system (Miltenyi Biotech, Bergisch Gladbach, Germany). In certain embodiments, CD40L fibroblasts are irradiated before use in the methods described herein. In one embodiment, B cells are cultured in the presence of one or more of IL-3, IL-7, Flt3 ligand, thrombopoietin, SCF, IL-2, IL-10, G-CSF and CpG. In certain embodiments, the methods include culturing the B cells in the presence of one or more of the aforementioned factors in conjunction with transformed stromal cells (*e.g.,* MS5) providing a low level of anchored CD40L and/or CD40L bound to a plate or a bead.

As discussed above, B cell activating factors induce expansion, proliferation, or differentiation of B cells. Accordingly, B cells are contacted with one or more B cell activating factors listed above to obtain an expanded cell population. A cell population may be expanded prior to transfection. Alternatively, or additionally, a cell population may be expanded following transfection. In one embodiment, expanding a B cell population comprises culturing cells with IL-2, IL-4, IL-10 and CD40L (see *e.g.,* Neron et al. PLoS ONE, 2012 7(12):e51946). In one embodiment, expanding a B cell population comprises culturing cells with IL-2, IL-10, CpG, and CD40L. In one embodiment, expanding a B cell population comprises culturing cells with IL-2, IL-4, IL-10, IL-15, IL-21, and CD40L. In one embodiment, expanding a B cell population comprises culturing cells with IL-2, IL-4, IL-10, IL-15, IL-21, and multimerized CD40L.

In another embodiment, expansion of a B cell population is induced and/or enhanced by a transgene introduced into the B cells. For example, a B cell that contains a recombinant receptor or an engineered receptor that induces a cell signaling pathway (*e.g.,* signaling downstream of CD40) upon binding its ligand (*e.g.,* a soluble ligand or a cell surface expressed ligand). In one embodiment, a B cell overexpresses CD40 due to expression of a CD40 transgene. In another embodiment, a B cell expresses an engineered receptor, including, *e.g.,* a recombinantly engineered antibody. In one embodiment, an engineered receptor is similar to a chimeric antigen receptor (CAR) and comprises a fusion protein of an scFv and an intracellular signaling portion of a B cell receptor (*e.g.,* CD40).

In one embodiment, expansion of a B cell population is induced and/or enhanced by a small molecule compound added to the cell culture. For example, a compound that binds to and dimerizes CD40 can be used to trigger the CD40 signaling pathway.

Any of a variety of culture media may be used in the present methods as would be known to the skilled person (see *e.g.,* Current Protocols in Cell Culture, 2000-2009 by John Wiley & Sons, Inc.). In one embodiment, media for use in the methods described herein includes, but is not limited to Iscove modified Dulbecco medium (with or without fetal bovine or other appropriate serum). Illustrative media also includes, but is not limited to, IMDM, RPMI 1640, AIM-V, DMEM, MEM, a-MEM, F-12, X-Vivo 15, and X-Vivo 20. In further embodiments, the medium may comprise a surfactant, an antibody, plasmanate or a reducing agent (*e.g*. N-acetyl-cysteine, 2-mercaptoethanol), one or more antibiotics, and/or additives such as insulin, transferrin, sodium selenite and cyclosporin. In some embodiments, IL-6, soluble CD40L, and a cross-linking enhancer may also be used.

B cells are cultured under conditions and for sufficient time periods to achieve differentiation and/or activation desired. In certain embodiments, the B cells are cultured under conditions and for sufficient time periods such that 10%, 15%, 20%, 25%, 30%, 35%, 40%, 45%, 50%, 55%, 60%, 65%, 70%, 75%, 80%, 85%, 90%, 95%, or even 100% of the B cells are differentiated and/or activated as desired. In one embodiment, the B cells are activated and differentiated into a mixed population of plasmablasts and plasma cells. As would be recognized by the skilled person, plasmablasts and plasma cells may be identified by cell surface protein expression patterns using standard flow cytometry methods as described elsewhere herein, such as expression of one or more of CD38, CD78, IL-6R, CD27^{high}, and CD138 and/or lack of, or reduction of, expression of one or more of CD19, CD20 and CD45. As would be understood by the skilled person, memory B cells are generally CD20+ CD19+ CD27+ CD38- while early plasmablasts are CD20- CD19+ CD27++ CD38++. In one embodiment, the cells cultured using the methods described herein are CD20-, CD38+, CD138-. In another embodiment, the cells have a phenotype of CD20-, CD38+, CD138+. In certain embodiments, cells are cultured for 1-7 days. In further embodiments, cells are cultured 7, 14, 21 days or longer. Thus, cells may be cultured under appropriate conditions for 1, 2, 3, 4, 5, 6, 7, 8, 9, 10, 11, 12, 13, 14, 15, 16, 17, 18, 19, 20, 21, 22, 23, 24, 25, 26, 27, 28, 29, or more days. Cells are re-plated, and media and supplements may be added or changed as needed using techniques known in the art.

In certain embodiments, the B cells are cultured under conditions and for sufficient time periods such that at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99% or 100% of the cells are differentiated and activated to produce Ig and/or to express the transgene.

The induction of B cell activation may be measured by techniques such as ³H-uridine incorporation into RNA (as B cells differentiate, RNA synthesis increases), or by ³H-thymidine incorporation, which measures DNA synthesis associated with cell proliferation. In one embodiment, interleukin-4 (IL-4) may be added to the culture medium at an appropriate concentration (*e.g*., about 10 ng/ml) for enhancement of B cell proliferation.

Alternatively, B cell activation is measured as a function of immunoglobulin secretion. For example, CD40L is added to resting B cells together with IL-4 (*e.g.,* 10 ng/ml) and IL-5 (*e.g.,* 5 ng/ml) or other cytokines that activate B cells. Flow cytometry may also be used for measuring cell surface markers typical of activated B cells. See *e.g.,* Civin CI, Loken MR, Int'l J. Cell Cloning 987; 5:1 -16; Loken, MR, et al, Flow Cytometry Characterization of Erythroid, Lymphoid and Monomyeloid Lineages in Normal Human Bone Marrow, in Flow Cytometry in Hematology, Laerum OD, Bjerksnes R. eds., Academic Press, New York 1992; pp. 31 -42; and LeBein TW, et al., Leukemia 1990; 4:354-358.

After culture for an appropriate period of time, such as, e.g., from 2, 3, 4, 5, 6, 7, 8, 9, or more days, generally around 3 days, an additional volume of culture medium may be added. Supernatant from individual cultures may be harvested at various times during culture and quantitated for IgM and IgG1 as described in Noelle et al., (1991) J. Immunol. 146:1118-1124. In one embodiment, the culture is harvested and measured for expression of the transgene of interest using flow cytometry, enzyme-linked immunosorbent assay (ELISA), ELISPOT or other assay known in the art.

In another embodiment, ELISA is used to measure antibody isotype production, *e.g.,* IgM, or a product of the transgene of interest. In certain embodiments, IgG determinations are made using commercially available antibodies, such as goat antihuman IgG, as capture antibody followed by detection using any of a variety of appropriate detection reagents such as biotinylated goat antihuman Ig, streptavidin alkaline phosphatase and substrate.

In certain embodiments, the B cells are cultured under conditions and for sufficient time periods such that the number of cells is 1, 10, 25, 50, 75, 100, 125, 150, 175, 200, 250, 300, 350, 400, 450, 500, 550, 600, 650, 700, 750, 800, 850, 900, 950, 1000 fold or more greater than the number of B cells at the start of culture. In one embodiment, the number of cells is 10-1000 fold greater, including consecutive integers therein, than the number of B cells at the start of culture. For example, an expanded B cell population is at least 10 fold greater than the initial isolated B cell population. In another embodiment, the expanded B cell population is at least 100 fold greater than the initial isolated B cell population. In one embodiment, the expanded B cell population is at least 500 fold greater than the initial isolated B cell population.

### Engineering of B cells

In various embodiments, the present disclosure relates to methods of transfecting, infecting, or otherwise incorporating into a B cell a transgene (e.g., a follistatin transgene), such that the transgene is expressed in the B cell. Any of the integration methods described herein or known in the art may in some embodiments be utilized for expressing follistatin in a B cell.

In one embodiment, the genetically modified B cells are transfected with a transgene. In particular embodiments, the genetically modified B cells are with a follistatin transgene.

Exemplary methods for transfecting B cells are provided in WO 2014/152832 and WO 2016/100932. Transfection of B cells may be accomplished using any of a variety of methods available in the art to introduce DNA or RNA into a B cell. Suitable techniques may include calcium phosphate transfection, DEAE-Dextran, electroporation, pressure-mediated transfection or "cell squeezing" (*e.g*., CellSqueeze microfluidic system, SQZ Biotechnologies), nano-particle-mediated or liposome-mediated transfection and transduction using retrovirus or other virus, *e.g*., vaccinia. See, *e.g*., Graham et al., 1973, Virology 52:456; Sambrook et al., 2001, Molecular Cloning, a Laboratory Manual, Cold Spring Harbor Laboratories; Davis et al., 1986, Basic Methods in Molecular Biology, Elsevier; Chu et al., 1981, Gene 13:197; US 5,124,259; US 5,297,983; US 5,283,185; US 5,661,018; US 6,878,548; US 7,799,555; US 8,551,780; and US 8,633,029. One example of a commercially available electroporation technique suitable for B cells is the Nucleofector^{™} transfection technology.

Transfection may take place prior to or during *in vitro* culture of the isolated B cells in the presence of one or more activating and/or differentiating factors described above. For example, cells are transfected on day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or 39 of *in vitro* culture. In one embodiment, cells are transfected on day 1, 2, or 3 of *in vitro* culture. In a particular embodiment, cells are transfected on day 2. For example, cells are electroporated on day 2 of *in vitro* culture for delivery of, *e.g.,* a plasmid, a transposon, a minicircle, or a self-replicating RNA. In another embodiment, cells are transfected on day 4, 5, 6, or 7 of *in vitro* culture. In a particular embodiment, cells are transfected on day 6 of *in vitro* culture. In another embodiment, cells are transfected on day 5 of *in vitro* culture.

In one embodiment, cells are transfected or otherwise engineered (e.g., via a targeted integration of a follistatin transgene) prior to activation. In another embodiment, cells are transfected or otherwise engineered (e.g., via a targeted integration of a follistatin transgene) during activation. In one embodiment, cells are transfected or otherwise engineered (e.g., via a targeted integration of a follistatin transgene) after activation. In one embodiment, cells are transfected or otherwise engineered (e.g., via a targeted integration of a follistatin transgene) prior to differentiation. In another embodiment, cells are transfected or otherwise engineered (e.g., via a targeted integration of a follistatin transgene) during differentiation. In one embodiment, cells are transfected or otherwise engineered (e.g., via a targeted integration of a follistatin transgene) after differentiation.

In one embodiment, a non-viral vector is used to deliver DNA or RNA (e.g., DNA or RNA comprising a sequence encoding a follistatin polypeptide) to memory B cells and/or plasma cells. For example, systems that may facilitate transfection of memory B cells and/or plasma cells without the need of a viral integration system include, without limitation, transposons (*e.g*., Sleeping Beauty or other transposon system such as Piggybac), zinc-finger nucleases (ZFNs), transcription activator-like effector nucleases (TALENs), clustered regularly interspaced short palindromic repeats (CRISPRs), meganucleases, minicircles, replicons, artificial chromosomes (*e.g.*, bacterial artificial chromosomes, mammalian artificial chromosomes, and yeast artificial chromosomes), plasmids, cosmids, and bacteriophage.

In some embodiments, such non-viral-dependent vector systems may also be delivered via a viral vector known in the art or described below. For example, in some embodiments, a viral vector (e.g., a retrovirus, lentivirus, adenovirus, adeno-associated virus), is utilized to deliver one or more non-viral vector (such as, e.g., one or more of the above-mentioned zinc-finger nucleases (ZFNs), transcription activator-like effector nucleases (TALENs), clustered regularly interspaced short palindromic repeats (CRISPRs) meganucleases, or any other enzyme / complementary vectors, polynucleotides, and/or polypeptides capable of facilitating the targeted integration. Accordingly, in some embodiments, a cell (e.g., B cells such as a memory B cells and/or plasma cells) may be engineered to express an exogenous sequence (e.g., a sequence encoding a follistatin polypeptide) via a targeted integration method. Such methods are known in the art and may comprise cleaving an endogenous locus in the cell using one or more nucleases (e.g., ZFNs, TALENs, CRISPR/Cas, meganuclease) and administering the follistatin transgene to the cell such that it is integrated into the endogenous locus and expressed in the cell. The follistatin transgene may be comprised in a donor sequence that is integrated into the host cell's DNA at or near the point of a cleavage by the nuclease.

The integration of the exogenous sequence (e.g., a sequence encoding a follistatin polypeptide) may occur via recombination. As would be clear to one of skill in the art, "Recombination" refers to a process of exchange of genetic information between two polynucleotides, including but not limited to, donor capture by non-homologous end joining (NHEJ) and homologous recombination. The recombination may be homologous recombination. For the purposes of this disclosure, "homologous recombination (HR)" refers to the specialized form of such exchange that takes place, for example, during repair of double-strand breaks in cells via homology-directed repair mechanisms. This process utilizes nucleotide sequence homology, whereby a "donor" molecule (e.g., donor polynucleotide sequence or donor vector comprising such a sequence) is utilized by a cell's DNA-repair machinery as a template to repair of a "target" molecule (*i.e.,* the one that experienced the double-strand break), and by these means causes the transfer of genetic information from the donor to the target. In some embodiments of HR-directed integration, the donor molecule may contain at least 2 regions of homology to the genome ("homology arms"). In some embodiments, the homology arms may be, e.g., of least 50-100 base pairs in length. The homology arms may have substantial DNA homology to a region of genomic DNA flanking the cleavage site wherein the targeted integration is to occur. The homology arms of the donor molecule may flank the DNA (e.g., comprising DNA encoding a follistatin) that is to be integrated into the target genome or target DNA locus. Breakage of the chromosome followed by repair using the homologous region of the plasmid DNA as a template may results in the transfer of the intervening transgene flanked by the homology arms into the genome. See, e.g., Koller et al. (1989) Proc. Nat'l. Acad. Sci. USA 86(22):8927-8931; Thomas et al. (1986) Cell 44(3):419-428. The frequency of this type of homology-directed targeted integration can be increased by up to a factor of 10⁵ by deliberate creation of a double-strand break in the vicinity of the target region (Hockemeyer et al. (2009) Nature Biotech. 27(9):851-857; Lombardo et al. (2007) Nature Biotech. 25(11):1298-1306; Moehle et al. (2007) Proc. Nat'l Acad. Sci. USA 104(9):3055-3060; Rouet et al. (1994) Proc. Nat'l Acad. Sci. USA 91(13):6064-6068.

Any nuclease capable of mediating the targeted cleavage of a genomic locus such that a transgene (e.g., a follistatin transgene) may be integrated into the genome of a target cell (e.g., by recombination such as HR) may be utilized in engineering a cell (e.g., a memory B cell or plasmablast) according to the present disclosure.

A double-strand break (DSB) or nick can be created by a site-specific nuclease such as a zinc-finger nuclease (ZFN), a TAL effector domain nuclease (TALEN), a meganuclease, or using the CRISPR/Cas9 system with an engineered crRNA/tract RNA (single guide RNA) to guide specific cleavage. See, for example, Burgess (2013) Nature Reviews Genetics 14:80-81, Urnov et al. (2010) Nature 435(7042):646-51; United States Patent Publications 20030232410; 20050208489; 20050026157; 20050064474; 20060188987; 20090263900; 20090117617; 20100047805; 20110207221; 20110301073 and International Publication WO 2007/014275.

In some embodiments, the cell (e.g., a memory B cell or a plasmablast) is engineered via Zinc Finger Nuclease-mediated targeted integration of a donor construct (e.g., a follistatin donor construct). A zinc finger nuclease (ZFN) is an enzyme that is able to recognize and cleave a target nucleotide sequence with specificity due to the coupling of a "zinc finger DNA binding protein" (ZFP) (or binding domain), which binds DNA in a sequence-specific manner through one or more zinc fingers, and a nuclease enzyme. ZFNs may comprise any suitable cleavage domains (e.g., a nuclease enzyme) operatively linked to a ZFP DNA-binding domain to form a engineered ZFN that can facilitate site-specific cleavage of a target DNA sequence (see, e.g., Kim et al. (1996) Proc Natl Acad Sci USA 93(3):1156-1160). For example, ZFNs may comprise a target-specific ZFP linked to a FOK1 enzyme or a portion of a FOK1 enzyme. In some embodiments, ZFN used in a ZFN-mediated targeted integration approach utilize two separate molecules, each comprising a subunit of a FOK1 enzyme each bound to a ZFP, each ZFP with specificity for a DNA sequence flanking a target cleavage site, and when the two ZFPs bind to their respective target DNA sites the FOK1 enzyme subunits are brought into proximity with one another and they bind together activating the nuclease activity which cleaves the target cleavage site. ZFNs have been used for genome modification in a variety of organisms (e.g., United States Patent Publications 20030232410; 20050208489; 20050026157; 20050064474; 20060188987; 20060063231; and International Publication WO 07/014,275) Custom ZFPs and ZFNs are commercially available from, e.g., Sigma Aldrich (St. Louis, MO), and any location of DNA may be routinely targeted and cleaved using such custom ZFNs.

**In** some embodiments, the cell (e.g., a memory B cell or a plasmablast) is engineered via CRISPR/Cas (e.g., CRISPR Cas9) Nuclease-mediated integration of a donor construct (e.g., a follistatin donor construct). A CRISPR (Clustered Regularly Interspaced Short Palindromic Repeats)/Cas (CRISPR Associated) nuclease system is an engineered nuclease system based on a bacterial system that may be used for genome engineering. It is based on part of the adaptive immune response of many bacteria and archea. When a virus or plasmid invades a bacterium, segments of the invader's DNA are converted into CRISPR RNAs (crRNA) by the 'immune' response. This crRNA then associates, through a region of partial complementarity, with another type of RNA called tracrRNA to guide the Cas9 nuclease to a region homologous to the crRNA in the target DNA called a "protospacer". Cas9 cleaves the DNA to generate blunt ends at the DSB at sites specified by a 20-nucleotide guide sequence contained within the crRNA transcript. Cas9 requires both the crRNA and the tracrRNA for site specific DNA recognition and cleavage. This system has now been engineered such that the crRNA and tracrRNA can be combined into one molecule (the "single guide RNA"), and the crRNA equivalent portion of the single guide RNA can be engineered to guide the Cas9 nuclease to target any desired sequence (see Jinek et al (2012) Science 337, p. 816-821, Jinek et al, (2013), eLife 2:e00471, and David Segal, (2013) eLife 2:e00563). Thus, the CRISPR/Cas system can be engineered to create a DSB at a desired target in a genome, and repair of the DSB can be influenced by the use of repair inhibitors to cause an increase in error prone repair. As will be clear to the skill artisan, other CRISPR nucleases, in addition to Cas9, are known and are suitable for use in the present invention.

In some embodiments, the CRISPR/Cas nuclease-mediated integration utilizes a Type II CRISPR. The Type II CRISPR is one of the most well characterized systems and carries out targeted DNA double-strand break in four sequential steps. First, two non-coding RNA, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Wastson-Crick base-pairing between the spacer on the crRNA and the protospacer on the target DNA next to a protospacer adjacent motif (PAM), an additional requirement for target recognition. Forth, Cas9 mediates cleavage of target DNA to create a double-stranded break within the protospacer.

The Cas9 related CRISPR/Cas system comprises two RNA non-coding components: tracrRNA and a pre-crRNA array containing nuclease guide sequences (spacers) interspaced by identical direct repeats (DRs). To use a CRISPR/Cas system to accomplish genome engineering, both functions of these RNAs must be present (see Cong et al, (2013) Sciencexpress 1/10.1126/science 1231143). In some embodiments, the tracrRNA and pre-crRNAs are supplied via separate expression constructs or as separate RNAs. In other embodiments, a chimeric RNA is constructed where an engineered mature crRNA (conferring target specificity) is fused to a tracrRNA (supplying interaction with the Cas9) to create a chimeric cr-RNA-tracrRNA hybrid (also termed a single guide RNA). (see Jinek ibid and Cong, ibid).

In some embodiments, a single guide RNA containing both the crRNA and tracrRNA may be engineered to guide the Cas9 nuclease to target any desired sequence (e.g., Jinek et al (2012) Science 337, p. 816-821, Jinek et al, (2013), eLife 2:e00471, David Segal, (2013) eLife 2:e00563). Thus, the CRISPR/Cas system may be engineered to create a DSB at a desired target in a genome.

Custom CRISPR/Cas systems are commercially available from, e.g., Dharmacon (Lafayette, CO), and any location of DNA may be routinely targeted and cleaved using such custom single guide RNA sequences. Single stranded DNA templates for recombination may be synthesized (e.g., via oligonucleotide synthesis methods known in the art and commercially available) or provided in a vector, e.g., a viral vector such as an AAV.

In some embodiments, the cell (e.g., a memory B cell or a plasmablast) is engineered via TALE-Nuclease (TALEN) mediated targeted integration of a donor construct (e.g., a follistatin donor construct). A "TALE DNA binding domain" or "TALE" is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein. TAL-effectors may contain a nuclear localization sequence, an acidic transcriptional activation domain and a centralized domain of tandem repeats where each repeat contains approximately 34 amino acids that are key to the DNA binding specificity of these proteins. (e.g., Schornack S, et al (2006) J Plant Physiol 163(3): 256-272). TAL effectors depend on the sequences found in the tandem repeats which comprises approximately 102 bp and the repeats are typically 91-100% homologous with each other (e.g., Bonas et al (1989) MoI Gen Genet 218: 127-136). These DNA binding repeats may be engineered into proteins with new combinations and numbers of repeats, to make artificial transcription factors that are able to interact with new sequences and activate the expression of a non-endogenous reporter gene (e.g., Bonas et al (1989) MoI Gen Genet 218: 127-136). Engineered TAL proteins may be linked to a FokI cleavage half domain to yield a TAL effector domain nuclease fusion (TALEN) to cleave target specific DNA sequence (e.g., Christian et al (2010) Genetics epub 10.1534/genetics.110.120717).

Custom TALEN are commercially available from, e.g., Thermo Fisher Scientific (Waltham, MA), and any location of DNA may be routinely targeted and cleaved.

In some embodiments, the cell (e.g., a memory B cell or a plasmablast) is engineered via Meganuclease-mediated targeted integration of a donor construct (e.g., a follistatin donor construct). A Meganuclease (or "homing endonuclease") is an endonuclease that binds and cleaves double-stranded DNA at a recognition sequence that is greater than 12 base pairs. Naturally occurring meganucleases may be monomeric (e.g., I-SceI) or dimeric (e.g., I-CreI). Naturally occurring meganucleases recognize 15-40 base-pair cleavage sites and are commonly grouped into four families: the LAGLIDADG family, the GIY-YIG family, the His-Cyst box family and the HNH family. Exemplary homing endonucleases include I-SceI, I-CeuI, PI-PspI, PI-Sce, I-SceIV, I-CsmI, I-PanI, I-SceII, I-PpoI, I-SceIII, I-CreI, I-TevI, I-TevII and I-TevIII. Their recognition sequences are known. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.. The term "Meganuclease" includes monomeric meganucleases, dimeric meganucleases and monomers that associate to form a dimeric meganucleases.

In certain embodiments, the methods and compositions described herein make use of a nuclease that comprises an engineered (non-naturally occurring) homing endonuclease (meganuclease). The recognition sequences of homing endonucleases and meganucleases such as I-SceI, I-CeuI, PI-PspI, PI-Sce, I-SceIV, I-CsmI, I-PanI, I-SceII, I-PpoI, I-SceIII, I-CreI, I-TevI, I-TevII and I-TevIII are known. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue. In addition, the DNA-binding specificity of homing endonucleases and meganucleases can be engineered to bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication No. 20070117128. The DNA-binding domains of the homing endonucleases and meganucleases may be altered in the context of the nuclease as a whole (*i.e*., such that the nuclease includes the cognate cleavage domain) or may be fused to a heterologous cleavage domain. Custom Meganuclease are commercially available from, e.g., New England Biolabs (Ipswich, MA), and any location of DNA may be routinely targeted and cleaved.

The engineering of the B cell may comprise administering one or more nucleases (e.g., ZFNs, TALENs, CRISPR/Cas, meganuclease) to a B cell, e.g., via one or more vectors encoding the nucleases, such that the vectors comprising the encoded nucleases are taken up by the B cell. The vectors may be viral vectors.

**In** some embodiments, the nucleases cleave a specific endogenous locus (e.g. safe harbor gene or locus of interest) in the cell (e.g., memory B cell or plasma cell) and one or more exogenous (donor) sequences (e.g., transgenes) are administered (e.g. one or more vectors comprising these exogenous sequences). In such embodiments, the donor sequence may encode follistatin (e.g., a follistatin transgene). The nuclease may induce a double-stranded (DSB) or single-stranded break (nick) in the target DNA. In some embodiments, targeted insertion of a donor transgene (e.g., a follistatin donor transgene) may be performed via homology directed repair (HDR), non-homology repair mechanisms (e.g., NHEJ- mediated end capture), or insertions and/or deletion of nucleotides (e.g. endogenous sequence) at the site of integration of a transgene (e.g., a follistatin transgene) into the cell's genome. In one embodiment, a method of transfecting a B cell comprises electroporating the B cell prior to contacting the B cell with a vector. In one embodiment, cells are electroporated on a day ranging from day 1 to day 12 of *in vitro* culture. In one embodiment, cells are electroporated on day 1, 2, 3, 4, 5, 6, 7, 8, or 9 of *in vitro* culture. In one embodiment, cells are electroporated on day 2 of *in vitro* culture for delivery of a plasmid.

In one embodiment, cells are transfected using a transposon. As used herein, the term "transposed" may in some embodiments refer to such a cell that is transfected with a transposon. Numerous transposon systems are known in the art and are suitable for use in the present invention. For example, the Sleeping Beauty transposon system and Piggybac transposon systems are well-known in the art and are suitable for use in the present invention. *See* e.g., Hackett P.B., et al., Evaluating Risks of Insertional Mutagenesis by DNA Transposons in Gene Therapy, Transl Res. 2013 April ; 161(4): 265-283; Hudecek M, et al., Going non-viral: the Sleeping Beauty transposon system breaks on through to the clinical side, Crit Rev Biochem Mol Biol. 2017 Aug;52(4):355-380. In some embodiments, cells are transfected using a Sleeping Beauty transposon. The Sleeping Beauty transposon may in some embodiments be a T2 Sleeping Beauty transposon or a T4 Sleeping Beauty transposon. In some embodiments, utilization of the Sleeping Beauty transposon system may comprise transfecting (e.g., via electroporation) B cells with a DNA construct encoding the transposon system machinery and a DNA construct encoding the follistatin polypeptide. In some embodiments, the DNA construct encoding the transposon system machinery may be pCMV-SB100x. In some embodiments, utilization of the Sleeping Beauty transposon system may comprise transfecting (e.g., via electroporation) B cells with a DNA construct encoding the follistatin polypeptide and further transfecting the B cells with mRNA encoding the transposon system machinery. In some embodiments, the mRNA encoding the transposon system machinery encodes the SB 100x transposase. In some embodiments, cells are transfected using a Piggybac transposon. In one embodiment, cells are transfected using a transposon (e.g., a T2 or T4 Sleeping beauty transposon or a Piggybac transposon) on a day ranging from day 1 to day 12 of *in vitro* culture. In one embodiment, cells are transfected using a transposon (e.g., a T2 or T4 Sleeping beauty transposon or a Piggybac transposon) on day 1, 2, 3, 4, 5, 6, 7, 8, or 9 of *in vitro* culture. In one embodiment, cells are transfected using a minicircle on a day ranging from day 1 to day 12 of *in vitro* culture. In one embodiment, cells are transfected using a minicircle on day 1, 2, 3, 4, 5, 6, 7, 8, or 9 of *in vitro* culture.

In one embodiment, cells are transfected using a Sleeping Beauty transposon (e.g., a T2 or T4 Sleeping beauty transposon) on a day ranging from day 1 to day 12 of *in vitro* culture. In one embodiment, cells are transduced using a Sleeping Beauty transposon system (e.g., a T2 or T4 Sleeping beauty transposon) on day 2 of *in vitro* culture via electroporation. In one embodiment, cells are transduced using a Sleeping Beauty transposon system (e.g., a T2 or T4 Sleeping beauty transposon) on day 5 of *in vitro* culture via electroporation. In one embodiment, cells are transduced using a Sleeping Beauty transposon system (e.g., a T2 or T4 Sleeping beauty transposon) on day 8 of *in vitro* culture via electroporation. In one embodiment, cells are transduced using a Sleeping Beauty transposon (e.g., a T2 or T4 Sleeping beauty transposon) system on day 11 of *in vitro* culture via electroporation. In one embodiment, cells are transduced using a Sleeping Beauty transposon (e.g., a T2 or T4 Sleeping beauty transposon) system on day 14 of *in vitro* culture via electroporation.

In one embodiment, cells are transfected using a Piggybac transposon on a day ranging from day 1 to day 12 of *in vitro* culture. In one embodiment, cells are transduced using a Piggybac transposon on day 2 of *in vitro* culture via electroporation. In one embodiment, cells are transduced using a Piggybac transposon on day 5 of *in vitro* culture via electroporation. In one embodiment, cells are transduced using a Piggybac transposon on day 8 of *in vitro* culture via electroporation. In one embodiment, cells are transduced using a Piggybac transposon on day 11 of *in vitro* culture via electroporation. In one embodiment, cells are transduced using a Piggybac transposon on day 14 of *in vitro* culture via electroporation.

**In** one embodiment, the B cells are contacted with a vector comprising a nucleic acid of interest operably linked to a promoter, under conditions sufficient to transfect at least a portion of the B cells. In one embodiment the B cells are contacted with a vector comprising a nucleic acid of interest operably linked to a promoter, under conditions sufficient to transfect at least 5% of the B cells. In a further embodiment, the B cells are contacted with a vector under conditions sufficient to transfect at least 5%, 10%, 15%, 20%, 25%, 30%, 35%, 40%, 45%, 50%, 55%, 60%, 70%, 75%, 80%, 85%, 90%, 95%, 96%, 97%, 98%, 99% or even 100% of the B cells. In one particular embodiment, the B cells, cultured *in vitro* as described herein, are transfected, in which case the cultured B cells are contacted with a vector as described herein under conditions sufficient to transfect at least 5%, 10% 15%, 20%, 25%, 30%, 35%, 40%, 45%, 50%, 55%, 60%, 70%, 75%, 80%, 85%, 90%, 95%, 96%, 97%, 98%, 99% or even 100% of the B cells.

Viral vectors may be employed to transduce memory B cells and/or plasma cells. Examples of viral vectors include, without limitation, adenovirus-based vectors, adeno-associated virus (AAV)-based vectors, retroviral vectors, retroviral-adenoviral vectors, and vectors derived from herpes simplex viruses (HSVs), including amplicon vectors, replication-defective HSV and attenuated HSV (see, *e.g*., Krisky, Gene Ther. 5: 1517-30, 1998; Pfeifer, Annu. Rev. Genomics Hum. Genet. 2:177-211, 2001).

In one embodiment, cells are transduced with a viral vector *(e.g.,* a lentiviral vector) on day 1, 2, 3, 4, 5, 6, 7, 8, or 9 of *in vitro* culture. In a particular embodiment, cells are transduced with a viral vector on day 5 of *in vitro* culture. In one embodiment, the viral vector is a lentivirus. In one embodiment, cells are transduced with a measles virus pseudotyped lentivirus on day 1 of *in vitro* culture.

In one embodiment, B cells are transduced with retroviral vectors using any of a variety of known techniques in the art (see, *e.g.,* Science 12 April 1996 272: 263-267; Blood 2007, 99:2342- 2350; Blood 2009, 1 13:1422-1431 ; Blood 2009 Oct 8; 1 14(15):3173-80; Blood. 2003;101 (6):2167-2174; Current Protocols in Molecular Biology or Current Protocols in Immunology, John Wiley & Sons, New York, N.Y.(2009)). Additional description of viral transduction of B cells may be found in WO 2011/085247 and WO 2014/152832.

For example, PBMCs, B- or T-lymphocytes from donors, and other B cell cancer cells such as B-CLLs may be isolated and cultured in IMDM medium or RPMI 1640 (GibcoBRL Invitrogen, Auckland, New Zealand) or other suitable medium as described herein, either serum-free or supplemented with serum (*e.g.,* 5-10% FCS, human AB serum, and serum substitutes) and penicillin/streptomycin and/or other suitable supplements such as transferrin and/or insulin. In one embodiment, cells are seeded at 1 x 10⁵ cells in 48-well plates and concentrated vector added at various doses that may be routinely optimized by the skilled person using routine methodologies. In one embodiment, B cells are transferred to an MS5 cell monolayer in RPMI supplemented with 10% AB serum, 5% FCS, 50ng/ml rhSCF, 10ng/ml rhlL-15 and 5ng/ml rhlL-2 and medium refreshed periodically as needed. As would be recognized by the skilled person, other suitable media and supplements may be used as desired.

Certain embodiments relate to the use of retroviral vectors, or vectors derived from retroviruses. "Retroviruses" are enveloped RNA viruses that are capable of infecting animal cells, and that utilize the enzyme reverse transcriptase in the early stages of infection to generate a DNA copy from their RNA genome, which is then typically integrated into the host genome. Examples of retroviral vectors Moloney murine leukemia virus (MLV)-derived vectors, retroviral vectors based on a Murine Stem Cell Virus, which provides long-term stable expression in target cells such as hematopoietic precursor cells and their differentiated progeny (see, *e.g*., Hawley et al., PNAS USA 93:10297-10302, 1996; Keller et al., Blood 92:877-887, 1998), hybrid vectors (see, *e.g.,* Choi, et al., Stem Cells 19:236-246, 2001), and complex retrovirus-derived vectors, such as lentiviral vectors.

**In** one embodiment, the B cells are contacted with a retroviral vector comprising a nucleic acid of interest operably linked to a promoter, under conditions sufficient to transduce at least a portion of the B cells. In one embodiment the B cells are contacted with a retroviral vector comprising a nucleic acid of interest operably linked to a promoter, under conditions sufficient to transduce at least 2% of the B cells. In a further embodiment, the B cells are contacted with a vector under conditions sufficient to transduce at least 2%, 3%, 4%, 5%, 10%, 15%, 20%, 25%, 30%, 35%, 40%, 45%, 50%, 55%, 60%, 70%, 75%, 80%, 85%, 90%, 95%, 96%, 97%, 98%, 99% or even 100% of the resting B cells. In one particular embodiment, the differentiated and activated B cells, cultured *in vitro* as described herein, are transduced, in which case the cultured differentiated/activated B cells are contacted with a vector as described herein under conditions sufficient to transduce at least 2%, 3%, 4%, 5%, 10% 15%, 20%, 25%, 30%, 35%, 40%, 45%, 50%, 55%, 60%, 70%, 75%, 80%, 85%, 90%, 95%, 96%, 97%, 98%, 99% or even 100% of the differentiated and activated B cells.

In certain embodiments, prior to transduction, the cells are prestimulated with Staphylococcus Aureus Cowan (SAC; Calbiochem, San Diego, CA) and/or IL-2 at appropriate concentrations known to the skilled person and routinely optimized. Other B cell activating factors (*e.g*., PMA), as are known to the skilled artisan and described herein may be used.

As noted above, certain embodiments employ lentiviral vectors. The term "lentivirus" refers to a genus of complex retroviruses that are capable of infecting both dividing and non-dividing cells. Examples of lentiviruses include HIV (human immunodeficiency virus; including HIV type 1, and HIV type 2), visna-maedi, the caprine arthritis-encephalitis virus, equine infectious anemia virus, feline immunodeficiency virus (FIV), bovine immune deficiency virus (BIV), and simian immunodeficiency virus (SIV). Lentiviral vectors can be derived from any one or more of these lentiviruses (see, *e.g*., Evans et al., Hum Gene Ther. 10:1479-1489, 1999; Case et al., PNAS USA 96:2988-2993, 1999; Uchida et al., PNAS USA 95:1 1939-1 1944, 1998; Miyoshi et al., Science 283:682-686, 1999; Sutton et al., J Virol 72:5781 -5788, 1998; and Frecha et al., Blood. 1 12:4843-52, 2008).

It has been documented that resting T and B cells can be transduced by a VSVG-coated LV carrying most of the HIV accessory proteins (vif, vpr, vpu, and nef) (see *e.g.,* Frecha et al., 2010 Mol. Therapy 18:1748). In certain embodiments the retroviral vector comprises certain minimal sequences from a lentivirus genome, such as the HIV genome or the SIV genome. The genome of a lentivirus is typically organized into a 5' long terminal repeat (LTR) region, the gag gene, the pol gene, the env gene, the accessory genes (*e.g.,* nef, vif, vpr, vpu, tat, rev) and a 3' LTR region. The viral LTR is divided into three regions referred to as U3, R (repeat) and U5. The U3 region contains the enhancer and promoter elements, the U5 region contains the polyadenylation signals, and the R region separates the U3 and U5 regions. The transcribed sequences of the R region appear at both the 5' and 3' ends of the viral RNA (see, *e.g.,* "RNA Viruses: A Practical Approach" (Alan J. Cann, Ed., Oxford University Press, 2000); O Narayan, J. Gen. Virology. 70:1617-1639, 1989; Fields et al., Fundamental Virology Raven Press., 1990; Miyoshi et al., J Virol. 72:8150-7,1998; and U.S. Pat. No. 6,013,516). Lentiviral vectors may comprise any one or more of these elements of the lentiviral genome, to regulate the activity of the vector as desired, or, they may contain deletions, insertions, substitutions, or mutations in one or more of these elements, such as to reduce the pathological effects of lentiviral replication, or to limit the lentiviral vector to a single round of infection.

Typically, a minimal retroviral vector comprises certain 5'LTR and 3'LTR sequences, one or more genes of interest (to be expressed in the target cell), one or more promoters, and a cis-acting sequence for packaging of the RNA. Other regulatory sequences can be included, as described herein and known in the art. The viral vector is typically cloned into a plasmid that may be transfected into a packaging cell line, such as a eukaryotic cell (*e.g.,* 293-HEK), and also typically comprises sequences useful for replication of the plasmid in bacteria.

In certain embodiments, the viral vector comprises sequences from the 5' and/or the 3' LTRs of a retrovirus such as a lentivirus. The LTR sequences may be LTR sequences from any lentivirus from any species. For example, they may be LTR sequences from HIV, SIV, FIV or BIV. Preferably the LTR sequences are HIV LTR sequences.

In certain embodiments, the viral vector comprises the R and U5 sequences from the 5' LTR of a lentivirus and an inactivated or "self-inactivating" 3' LTR from a lentivirus. A "self-inactivating 3' LTR" is a 3' long terminal repeat (LTR) that contains a mutation, substitution or deletion that prevents the LTR sequences from driving expression of a downstream gene. A copy of the U3 region from the 3' LTR acts as a template for the generation of both LTR's in the integrated provirus. Thus, when the 3' LTR with an inactivating deletion or mutation integrates as the 5' LTR of the provirus, no transcription from the 5' LTR is possible. This eliminates competition between the viral enhancer/promoter and any internal enhancer/promoter. Self-inactivating 3' LTRs are described, for example, in Zufferey et al., J Virol. 72:9873-9880, 1998; Miyoshi et al., J Virol. 72:8150-8157, 1998; and Iwakuma et al., J Virology 261: 120-132, 1999. Self-inactivating 3' LTRs may be generated by any method known in the art. In certain embodiments, the U3 element of the 3' LTR contains a deletion of its enhancer sequence, preferably the TATA box, Spl and/or NF-kappa B sites. As a result of the self-inactivating 3' LTR, the provirus that is integrated into the host cell genome will comprise an inactivated 5' LTR.

The vectors provided herein typically comprise a gene that encodes a protein, e.g., follistatin, that is desirably expressed in one or more target cell. But, the vectors provided herein may also comprise genes that encode other molecules, (such as, e.g., siRNA) that are desirably expressed in one or more target cells. In some embodiments, in a viral vector, the gene of interest (e.g., follistatin) is preferably located between the 5' LTR and 3' LTR sequences. Further, in some embodiments, the gene of interest (e.g., follistatin) is preferably in a functional relationship with other genetic elements, for example, transcription regulatory sequences such as promoters and/or enhancers, to regulate expression of the gene of interest (e.g., follistatin) in a particular manner once the gene is incorporated into the target cell. In certain embodiments, the useful transcriptional regulatory sequences are those that are highly regulated with respect to activity, both temporally and spatially.

In certain embodiments, one or more additional genes may be incorporated as a safety measure, e.g., to allow for the selective killing of or depleting transfected target cells within a heterogeneous population, such as within a human patient. In one non-limiting exemplary embodiment, the gene is a thymidine kinase gene (TK), the expression of which renders a target cell susceptible to the action of the drug gancyclovir. In some embodiments, the additional gene is a cell surface protein tag. In some embodiments, the gene is a suicide gene. In some embodiments, the suicide gene is a caspase 9 suicide gene activated by a dimerizing drug (see, *e.g.,* Tey et al., Biology of Blood and Marrow Transplantation 13:913-924, 2007).

In certain embodiments, one or more additional genes encoding a marker protein may be placed before or after the primary gene (e.g., a follistatin gene) in a viral or non-viral vector to allow for identification and/or selection of cells that are expressing the desired protein (e.g., follistatin). Certain embodiments incorporate an additional cell surface protein that may facilitate identification and/or selection of cells that are expressing the desired protein (e.g., follistatin). Certain embodiments incorporate a fluorescent marker protein, such as green fluorescent protein (GFP) or red fluorescent protein (RFP), along with the primary gene of interest (e.g., a follistatin gene). If one or more additional reporter genes are included, IRES sequences or 2A elements may also be included, separating the primary gene of interest (e.g., a follistatin gene) from a reporter gene and/or any other gene of interest.

Certain embodiments may employ genes that encode one or more selectable markers. Examples include selectable markers that are effective in a eukaryotic cell or a prokaryotic cell, such as a gene for a drug resistance that encodes a factor necessary for the survival or growth of transformed host cells grown in a selective culture medium. Exemplary selection genes encode proteins that confer resistance to antibiotics or other toxins, *e.g*., G418, hygromycin B, puromycin, zeocin, ouabain, blasticidin, ampicillin, neomycin, methotrexate, or tetracycline, complement auxotrophic deficiencies, or supply may be present on a separate plasmid and introduced by co-transfection with the viral vector. In one embodiment, the gene encodes for a mutant dihydrofolate reductase (DHFR) that confers methotrexate resistance. Certain other embodiments may employ genes that encode one or cell surface receptors that can be used for tagging and detection or purification of transfected cells (*e*.*g*., low-affinity nerve growth factor receptor (LNGFR) or other such receptors useful as transduction tag systems. See *e.g.,* Lauer et al., Cancer Gene Ther. 2000 Mar;7(3):430-7.

Certain viral vectors such as retroviral vectors employ one or more heterologous promoters, enhancers, or both. In certain embodiments, the U3 sequence from a retroviral or lentiviral 5' LTR may be replaced with a promoter or enhancer sequence in the viral construct. Certain embodiments employ an "internal" promoter/enhancer that is located between the 5' LTR and 3' LTR sequences of the viral vector, and is operably linked to the gene of interest (e.g., a follistatin gene).

A "functional relationship" and "operably linked" mean, without limitation, that the gene (e.g., a follistatin gene) is in the correct location and orientation with respect to the promoter and/or enhancer, such that expression of the gene (e.g., a follistatin gene) will be affected when the promoter and/or enhancer is contacted with the appropriate regulatory molecules. Any enhancer/promoter combination may be used that either regulates (*e.g*., increases, decreases) expression of the viral RNA genome in the packaging cell line, regulates expression of the selected gene of interest in an infected target cell, or both.

A promoter is an expression control element formed by a DNA sequence that permits polymerase binding and transcription to occur. Promoters are untranslated sequences that are located upstream (5') of the start codon of a selected gene of interest (typically within about 100 to 1000 bp) and control the transcription and translation of the coding polynucleotide sequence to which they are operably linked. Promoters may be inducible or constitutive. Inducible promoters initiate increased levels of transcription from DNA under their control in response to some change in culture conditions, such as a change in temperature. Promoters may be unidirectional or bidirectional. Bidirectional promoters can be used to co-express two genes, *e.g.,* a gene of interest such as follistatin and a selection marker. Alternatively, a bidirectional promoter configuration comprising two promoters, each controlling expression of a different gene, in opposite orientation in the same vector may be utilized.

A variety of promoters are known in the art, as are methods for operably linking the promoter to the polynucleotide coding sequence. Both native promoter sequences and many heterologous promoters may be used to direct expression of the selected gene of interest. Certain embodiments employ heterologous promoters, because they generally permit greater transcription and higher yields of the desired protein as compared to the native promoter.

Certain embodiments may employ heterologous viral promoters. Examples of such promoters include those obtained from the genomes of viruses such as polyoma virus, fowlpox virus, adenovirus, bovine papilloma virus, avian sarcoma virus, cytomegalovirus, a retrovirus, hepatitis-B virus and Simian Virus 40 (SV40). Certain embodiments may employ heterologous mammalian promoter, such as the actin promoter, an immunoglobulin promoter, a heat-shock promoter, or a promoter that is associated with the native sequence of the gene of interest (e.g., a follistatin gene). Typically, the promoter is compatible with the target cell, such as an activated B-lymphocyte, a plasma B cell, a memory B cell or other lymphocyte target cell.

Certain embodiments may employ one or more of the RNA polymerase II and III promoters. A suitable selection of RNA polymerase III promoters can be found, for example, in Paule and White. Nucleic Acids Research., Vol. 28, pp 1283-1298, 2000. RNA polymerase II and III promoters also include any synthetic or engineered DNA fragments that can direct RNA polymerase II or III, respectively, to transcribe its downstream RNA coding sequences. Further, the RNA polymerase II or III (Pol II or III) promoter or promoters used as part of the viral vector can be inducible. Any suitable inducible Pol II or III promoter can be used with the methods described herein. Exemplary Pol II or III promoters include the tetracycline responsive promoters provided in Ohkawa and Taira, Human Gene Therapy, Vol. 11, pp 577-585, 2000; and Meissner et al., Nucleic Acids Research, Vol. 29, pp 1672-1682, 2001.

Non-limiting examples of constitutive promoters that may be used include the promoter for ubiquitin, the CMV promoter (see, *e.g*., Karasuyama et al., J. Exp. Med. 169:13, 1989), the β-actin (see, *e.g*., Gunning et al., PNAS USA 84:4831 -4835, 1987), the elongation factor-1 alpha (EF-1 alpha) promoter, the CAG promoter, and the pgk promoter (see, *e.g.,* Adra et al., Gene 60:65-74, 1987); Singer-Sam et al., Gene 32:409-417, 1984; and Dobson et al., Nucleic Acids Res. 10:2635-2637, 1982). Non-limiting examples of tissue specific promoters include the lck promoter (see, *e.g.,* Garvin et al., Mol. Cell Biol. 8:3058-3064, 1988; and Takadera et al., Mol. Cell Biol. 9:2173-2180, 1989), the myogenin promoter (Yee et al., Genes and Development 7:1277-1289. 1993), and the thyl promoter (see, *e.g*., Gundersen et al., Gene 1 13:207-214, 1992).

Additional examples of promoters include the ubiquitin-C promoter, the human µ heavy chain promoter or the Ig heavy chain promoter (*e.g*., MH), and the human κ light chain promoter or the Ig light chain promoter (*e.g*., EEK), which are functional in B-lymphocytes. The MH promoter contains the human µ heavy chain promoter preceded by the iEµ enhancer flanked by matrix association regions, and the EEK promoter contains the κ light chain promoter preceded an intronic enhancer (iEκ), a matrix associated region, and a 3' enhancer (3Ex) (see, *e.g.,* Luo et al., Blood. 1 13:1422-1431, 2009, and U.S. Patent Application Publication No. 2010/0203630). Accordingly, certain embodiments may employ one or more of these promoter or enhancer elements.

In one embodiment, one promoter drives expression of a selectable marker and a second promoter drives expression of the gene of interest (e.g., a follistatin gene). For example, in one embodiment, the EF-1 alpha promoter drives the production of a selection marker *(e.g.,* DHFR) and a miniature CAG promoter (see, *e.g.,* Fan et al. Human Gene Therapy 10:2273-2285, 1999) drives expression of the gene of interest (*e.g*., follistatin).

As noted above, certain embodiments employ enhancer elements, such as an internal enhancer, to increase expression of the gene of interest. Enhancers are cis-acting elements of DNA, usually about 10 to 300 bp in length, that act on a promoter to increase its transcription. Enhancer sequences may be derived from mammalian genes (*e.g.,* globin, elastase, albumin, α-fetoprotein, insulin), such as the 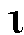 εµ enhancer, the 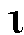 εκ intronic enhancer, and the 3' εκ enhancer. Also included are enhancers from a eukaryotic virus, including the SV40 enhancer on the late side of the replication origin (bp 100-270), the cytomegalovirus early promoter enhancer, the polyoma enhancer on the late side of the replication origin, and adenovirus enhancers. Enhancers may be spliced into the vector at a position 5' or 3' to the antigen-specific polynucleotide sequence, but are preferably located at a site 5' from the promoter. Persons of skill in the art will select the appropriate enhancer based on the desired expression pattern.

In certain embodiments, promoters are selected to allow for inducible expression of the gene of interest (e.g., a follistatin gene). A number of systems for inducible expression are known in the art, including the tetracycline responsive system and the lac operator-repressor system. It is also contemplated that a combination of promoters may be used to obtain the desired expression of the gene of interest (e.g., a follistatin gene). The skilled artisan will be able to select a promoter based on the desired expression pattern of the gene in the organism and/or the target cell of interest.

Certain viral vectors contain cis-acting packaging sequences to promote incorporation of the genomic viral RNA into the viral particle. Examples include psi-sequences. Such cis-acting sequences are known in the art. In certain embodiments, the viral vectors described herein may express two or more genes, which may be accomplished, for example, by incorporating an internal promoter that is operably linked to each separate gene beyond the first gene, by incorporating an element that facilitates co-expression such as an internal ribosomal entry sequence (IRES) element (U.S. Pat. No. 4,937,190) or a 2A element, or both. Merely by way of illustration, IRES or 2A elements may be used when a single vector comprises sequences encoding each chain of an immunoglobulin molecule with a desired specificity. For instance, the first coding region (encoding either the heavy or light chain) may be located immediately downstream from the promoter, and the second coding region (encoding the other chain) may be located downstream from the first coding region, with an IRES or 2A element located between the first and second coding regions, preferably immediately preceding the second coding region. In other embodiments, an IRES or 2A element is used to co-express an unrelated gene, such as a reporter gene, a selectable marker, a cell surface protein, or a gene that enhances immune function. Examples of IRES sequences that can be used include, without limitation, the IRES elements of encephalomyelitis virus (EMCV), foot-and- mouth disease virus (FMDV), Theiler's murine encephalomyelitis virus (TMEV), human rhinovirus (HRV), coxsackievirus (CSV), poliovirus (POLIO), Hepatitis A virus (HAV), Hepatitis C virus (HCV), and Pestiviruses (*e.g*., hog cholera virus (HOCV) and bovine viral diarrhea virus (BVDV)) (see, *e.g.,* Le et al., Virus Genes 12:135-147, 1996; and Le et al., Nuc. Acids Res. 25:362-369, 1997). One example of a 2A element includes the F2A sequence from foot-and-mouth disease virus.

In certain embodiments, the vectors provided herein also contain additional genetic elements to achieve a desired result. For example, certain viral vectors may include a signal that facilitates nuclear entry of the viral genome in the target cell, such as an HIV-1 flap signal. As a further example, certain viral vectors may include elements that facilitate the characterization of the provirus integration site in the target cell, such as a tRNA amber suppressor sequence. Certain viral vectors may contain one or more genetic elements designed to enhance expression of the gene of interest (e.g., a follistatin gene). For example, a woodchuck hepatitis virus responsive element (WRE) may be placed into the construct (see, *e.g.,* Zufferey et al., J. Virol. 74:3668-3681, 1999; and Deglon et al., Hum. Gene Ther. 11:179-190, 2000). As another example, a chicken β-globin insulator may also be included in the construct. This element has been shown to reduce the chance of silencing the integrated DNA in the target cell due to methylation and heterochromatinization effects. In addition, the insulator may shield the internal enhancer, promoter and exogenous gene from positive or negative positional effects from surrounding DNA at the integration site on the chromosome. Certain embodiments employ each of these genetic elements. In another embodiment, the viral vectors provided herein may also contain a Ubiquitous Chromatin Opening Element (UCOE) to increase expression (see *e.g.,* Zhang F, et al., Molecular Therapy: The journal of the American Society of Gene Therapy 2010 Sep;18(9):1640-9.)

In certain embodiments, the viral vectors (*e.g*., retroviral, lentiviral) provided herein are "pseudo-typed" with one or more selected viral glycoproteins or envelope proteins, mainly to target selected cell types. Pseudo-typing refers to generally to the incorporation of one or more heterologous viral glycoproteins onto the cell-surface virus particle, often allowing the virus particle to infect a selected cell that differs from its normal target cells. A "heterologous" element is derived from a virus other than the virus from which the RNA genome of the viral vector is derived. Typically, the glycoprotein-coding regions of the viral vector have been genetically altered such as by deletion to prevent expression of its own glycoprotein. Merely by way of illustration, the envelope glycoproteins gp41 and/or gp120 from an HIV-derived lentiviral vector are typically deleted prior to pseudo-typing with a heterologous viral glycoprotein.

In certain embodiments, the viral vector is pseudo-typed with a heterologous viral glycoprotein that targets B lymphocytes. In certain embodiments, the viral glycoprotein allows selective infection or transduction of resting or quiescent B lymphocytes. In certain embodiments, the viral glycoprotein allows selective infection of B lymphocyte plasma cells, plasmablasts, and activated B cells. In certain embodiments, the viral glycoprotein allows infection or transduction of quiescent B lymphocytes, plasmablasts, plasma cells, and activated B cells. In certain embodiments, viral glycoprotein allows infection of B cell chronic lymphocyte leukemia cells. In one embodiment, the viral vector is pseudo-typed with VSV-G. In another embodiment, the heterologous viral glycoprotein is derived from the glycoprotein of the measles virus, such as the Edmonton measles virus. Certain embodiments pseudo-type the measles virus glycoproteins hemagglutinin (H), fusion protein (F), or both (see, *e.g.,* Frecha et al., Blood. 1 12:4843-52, 2008; and Frecha et al., Blood. 1 14:3173-80, 2009). In one embodiment, the viral vector is pseudo-typed with gibbon ape leukemia virus (GALV). In one embodiment, the viral vector is pseudo-typed with cat endogenous retrovirus (RD114). In one embodiment, the viral vector is pseudo-typed with baboon endogenous retrovirus (BaEV). In one embodiment, the viral vector is pseudo-typed with murine leukemia virus (MLV). In one embodiment, the viral vector is pseudo-typed with gibbon ape leukemia virus (GALV). In further embodiments, the viral vector comprises an embedded antibody binding domain, such as one or more variable regions (e.g., heavy and light chain variable regions) which serves to target the vector to a particular cell type.

Generation of viral vectors can be accomplished using any suitable genetic engineering techniques known in the art, including, without limitation, the standard techniques of restriction endonuclease digestion, ligation, transformation, plasmid purification, PCR amplification, and DNA sequencing, for example as described in Sambrook et al. (Molecular Cloning: A Laboratory Manual. Cold Spring Harbor Laboratory Press, N.Y. (1989)), Coffin et al. (Retroviruses. Cold Spring Harbor Laboratory Press, N.Y. (1997)) and "RNA Viruses: A Practical Approach" (Alan J. Cann, Ed., Oxford University Press, (2000)).

Any variety of methods known in the art may be used to produce suitable retroviral particles whose genome comprises an RNA copy of the viral vector. As one method, the viral vector may be introduced into a packaging cell line that packages the viral genomic RNA based on the viral vector into viral particles with a desired target cell specificity. The packaging cell line typically provides in trans the viral proteins that are required for packaging the viral genomic RNA into viral particles and infecting the target cell, including the structural gag proteins, the enzymatic pol proteins, and the envelope glycoproteins.

In certain embodiments, the packaging cell line stably expresses certain necessary or desired viral proteins (*e.g*., gag, pol) (see, *e.g*., U.S. Pat. No. 6,218,181). In certain embodiments, the packaging cell line is transiently transfected with plasmids that encode certain of the necessary or desired viral proteins (*e.g*., gag, pol, glycoprotein), including the measles virus glycoprotein sequences described herein. In one exemplary embodiment, the packaging cell line stably expresses the gag and pol sequences, and the cell line is then transfected with a plasmid encoding the viral vector and a plasmid encoding the glycoprotein. Following introduction of the desired plasmids, viral particles are collected and processed accordingly, such as by ultracentrifugation to achieve a concentrated stock of viral particles. Exemplary packaging cell lines include 293 (ATCC CCL X), HeLa (ATCC CCL 2), D17 (ATCC CCL 183), MDCK (ATCC CCL 34), BHK (ATCC CCL-10) and Cf2Th (ATCC CRL 1430) cell lines.

### Therapeutic Agent

As used herein "gene of interest" or "gene" or "nucleic acid of interest" refers to a transgene to be expressed in the target transfected cell. In particular embodiments, the gene is a follistatin gene. While the term "gene" may be used, this is not to imply that this is a gene as found in genomic DNA and is used interchangeably with the term "nucleic acid". Generally, the nucleic acid of interest provides suitable nucleic acid for encoding a therapeutic agent (e.g., follistatin) and may comprise cDNA or DNA and may or may not include introns, but generally does not include introns. As noted elsewhere, the nucleic acid of interest is operably linked to expression control sequences to effectively express the protein of interest in the target cell. In certain embodiments, the vectors described herein may comprise one or more genes of interest, and may include 2, 3, 4, or 5 or more genes of interest, such as for example, the heavy and light chains of an immunoglobulin that may be organized using an internal promoter as described herein.

The recitation "polynucleotide" or "nucleic acid" as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA and RNA. The nucleic acid or gene of interest may be any nucleic acid encoding a protein of interest.

In some embodiments, any one of the embodiments disclosed herein may utilize a gene of interest that is a follistatin protein. The follistatin protein may, in some embodiments, be any one of the follistatin proteins set forth in SEQ ID NOS: 1-4. Thus, in some embodiments, the therapeutic agent delivered to the genetically modified B cell, as described herein, may be a follistatin protein.

### Follistatin

In various aspects, the present disclosure relates to B cells engineered to express follistatin (e.g., one or more follistatin polypeptides). As used herein, the term "follistatin" refers to a family of follistatin (FST) proteins and follistatin-related proteins, derived from any species. Follistatin is an autocrine glycoprotein that is expressed in nearly all tissues of higher animals. It was initially isolated from follicular fluid and was identified as a protein fraction that inhibited follicle-stimulating hormone (FSH) secretion from the anterior pituitary, and therefore was designated as FSH-suppressing protein (FSP). Subsequently, its primary function has been determined to be the binding and neutralization of members of the TGF-β superfamily including, for example, activin, a paracrine hormone that enhances secretion of FSH in the anterior pituitary.

In some embodiments, the terms "follistatin polypeptide" or "a follistatin" are used to refer to polypeptides comprising any naturally occurring polypeptide of the follistatin family as well as any variants thereof (including mutants, fragments, fusions, and peptidomimetic forms) that retain a useful activity, including, for example, ligand binding (e.g., myostatin, GDF-11, activin A, activin B) or heparin binding. For example, in some embodiments, follistatin polypeptides may include polypeptides comprising an amino acid sequence derived from the sequence of any known follistatin having a sequence at least about 80% identical to the sequence of a follistatin polypeptide, and preferably at least 85%, 90%, 95%, 97%, 99% or greater identity.

Follistatin is a single-chain polypeptide with a range of molecular weights from 31 to 49 kDa based on alternative mRNA splicing and variable glycosylation of the protein. The human gene encoding follistatin (FST) has six exons spanning 5329 bp on chromosome 5q11.2 and gives rise to two main transcripts: transcript variant FST344 (1122 bp) and transcript FST317 (1386 bp). Exon 1 in FST encodes the follistatin signal peptide, exon 2 encodes the follistatin N-terminal domain, and each of exons 3-5 encode a follistatin module. Due to alternative splicing, either one of exon 6A (which codes for an acidic region in FST344) or exon 6B (which contains two bases of the stop codon of FST317) are utilized (Shimasaki, S. et al., 1988).

These alternatively spliced mRNAs (FST344 and FST317) result in the production of two follistatin proteins of 315 amino acids *(i.e.,* FST315) and 288 amino acids *(i.e.,* FST288), respectively, after removal of the 29 amino acid signal peptide, and follistatin 315 can be further proteolytically degraded to follistatin 303 (FST303). Analysis of the amino acid sequence has revealed that the native human follistatin polypeptide comprises five domains (from the N-terminal side): a signal sequence peptide (amino acids 1-29 of SEQ ID NO:1), an N-terminal domain (FSN) (amino acids 30-94 of SEQ ID NO:1), follistatin domain I (FSDI) (amino acids 95-164 of SEQ ID NO:1), follistatin domain II (FSDII) (amino acids (168-239 of SEQ ID NO:1), and follistatin domain III (FSDIII) (amino acids 245-316 of SEQ ID NO:1). *See* PNAS, U.S.A., 1988, Vol. 85, No 12, pp 4218-4222.

The human follistatin-288 (FST288) precursor (*i.e*., FST317) has the following amino acid sequence, with the signal peptide indicated in bold, the N-terminal domain (FSN) indicated by single underlining, and the follistatin domains I-III (FSI, FSII, FSIII) indicated by double underlining.

MVRARHQPGGLCLLLLLLCQFMEDRSAQAGNCWLRQAKNGRCQVLYKTEL
SKEECCSTGRLSTSWTEEDVNDNTLFKWMIFNGGAPNCIPCKETCENVDC
GPGKKCRMNKKNKPRCVCAPDCSNITWKGPVCGLDGKTYRNECALLKARC
KEQPELEVQYQGRCKKTCRDVFCPGSSTCVVDQTNNAYCVTCNRICPEPA
SSEQYLCGNDGVTYSSACHLRKATCLLGRSIGLAYEGKCIKAKSCEDIQC
TGGKKCLWDFKVGRGRCSLCDELCPDSKSDEPVCASDNATYASECAMKEA
ACSSGVLLEVKHSGSCN (SEQ ID NO: 1)

The processed (mature) human follistatin variant (FST288) has the following amino acid sequence with the N-terminal domain indicated by single underlining, and the follistatin domains I-III indicated by double underlining. Moreover, it will be appreciated that any of the initial amino acids G or N, prior to the first cysteine may be removed by processing or intentionally eliminated without any consequence, and polypeptides comprising such slightly smaller polypeptides are further included.

GNCWLRQAKNGRCQVLYKTELSKEECCSTGRLSTSWTEEDVNDNTLFKWM
IFNGGAPNCIPCKETCENVDCGPGKKCRMNKKNKPRCVCAPDCSNITWKG
PVCGLDGKTYRNECALLKARCKEQPELEVQYQGRCKKTCRDVFCPGSSTC
VVDQTNNAYCVTCNRICPEPASSEQYLCGNDGVTYSSACHLRKATCLLGR
SIGLAYEGKCIKAKSCEDIQCTGGKKCLWDFKVGRGRCSLCDELCPDSKS
DEPVCASDNATYASECAMKEAACSSGVLLEVKHSGSCN (SEQ ID NO: 2)

The human follistatin-315 (FST315) precursor (*i.e*., FST344) has the following amino acid sequence, with the signal peptide indicated in bold, the N-terminal domain (FSN) indicated by single underlining, and the follistatin domains I-III (FSI, FSII, FSIII) indicated by double underlining (NCBI Accession Number AAH04107.1; 344 amino acids).

MVRARHQPGGLCLLLLLLCQFMEDRSAQAGNCWLRQAKNGRCQVLYKTEL
SKEECCSTGRLSTSWTEEDVNDNTLFKWMIFNGGAPNCIPCKETCENVDC
GPGKKCRMNKKNKPRCVCAPDCSNITWKGPVCGLDGKTYRNECALLKARC
KEQPELEVQYQGRCKKTCRDVFCPGSSTCVVDQTNNAYCVTCNRICPEPA
SSEQYLCGNDGVTYSSACHLRKATCLLGRSIGLAYEGKCIKAKSCEDIQC
TGGKKCLWDFKVGRGRCSLCDELCPDSKSDEPVCASDNATYASECAMKEA
ACSSGVLLEVKHSGSCNSISEDTEEEEEDEDQDYSFPISSILEW
(SEQ ID NO: 3)

The processed (mature) human FST315 has the following amino acid sequence with the N-terminal domain indicated by single underlining, and the follistatin domains I-III indicated by double underlining. Moreover, it will be appreciated that any of the initial amino acids G or N, prior to the first cysteine may be removed by processing or intentionally eliminated without any consequence, and polypeptides comprising such slightly smaller polypeptides are further included.

GNCWLRQAKNGRCQVLYKTELSKEECCSTGRLSTSWTEEDVNDNTLFKWM
IFNGGAPNCIPCKETCENVDCGPGKKCRMNKKNKPRCVCAPDCSNITWKG
PVCGLDGKTYRNECALLKARCKEQPELEVQYQGRCKKTCRDVFCPGSSTC
VVDQTNNAYCVTCNRICPEPASSEQYLCGNDGVTYSSACHLRKATCLLGR
SIGLAYEGKCIKAKSCEDIQCTGGKKCLWDFKVGRGRCSLCDELCPDSKS
DEPVCASDNATYASECAMKEAACSSGVLLEVKHSGSCNSISEDTEEEEED
EDQDYSFPISSILEW (SEQ ID NO: 4)

Follistatin polypeptides of the disclosure may include any naturally occurring domain of a follistatin protein as well as variants thereof (e.g., mutants, fragments, and peptidomimetic forms) that retain a useful activity. For example, it is well-known that FST315 and FST288 have high affinity for both activin (activin A and activin B) and myostatin (and the closely related GDF11) and that the follistatin domains (e.g., FSN and FSD I-III) are thought to be involved in the binding of such TGF-β ligands. However, it believed that each of these three domains may have a different affinity for these TGF-β ligands. For example, a study has demonstrated that polypeptide constructs comprising only the N-terminal domain (FSN) and two FSDI domains in tandem retained high affinity for myostatin, demonstrated little or no affinity for activin and promoted systemic muscle growth when introduced into a mouse by gene expression (Nakatani et al., The FASEB Journal, Vol. 22477-487 (2008)).

Accordingly, the present disclosure encompasses, in part, variant follistatin proteins that demonstrate selective binding and/or inhibition of a given TGF-β ligand relative to the naturally occurring FST protein (e.g., maintaining high-affinity for myostain while having a significantly reduced affinity for activin).

Thus, this disclosure provides polynucleotides (isolated or purified or pure polynucleotides) encoding therapeutic agents (e.g., follistatin) of this disclosure for genetically modifying B cells, vectors (including cloning vectors and expression vectors) comprising such polynucleotides, and cells (*e.g*., host cells) transformed or transfected with a polynucleotide or vector according to this disclosure. In certain embodiments, any one of the embodiments disclosed in the present disclosure may utilize a follistatin (e.g., for expression in a B cell) that is selected from the follistatin polypeptides of SEQ ID NOS: 1-4. In certain embodiments, any one of the embodiments disclosed in the present disclosure may utilize a follistatin (e.g., for expression in a B cell) that a human follistatin FST344 splice site variant. In certain embodiments, a polynucleotide (DNA or RNA) encoding a protein of interest (e.g., a follistatin) of this disclosure is contemplated. Expression cassettes encoding proteins of interest are also contemplated herein.

The present disclosure also relates to vectors that include a polynucleotide of this disclosure and, in particular, to recombinant expression constructs. In one embodiment, this disclosure contemplates a vector comprising a polynucleotide encoding a protein of this disclosure (e.g., follistatin), along with other polynucleotide sequences that cause or facilitate transcription, translation, and processing of such a protein-encoding sequences. Appropriate cloning and expression vectors for use with prokaryotic and eukaryotic hosts are described, for example, in Sambrook et al, Molecular Cloning: A Laboratory Manual, Second Edition, Cold Spring Harbor, NY, (1989). Exemplary cloning/expression vectors include cloning vectors, shuttle vectors, and expression constructs, that may be based on plasmids, phagemids, phasmids, cosmids, viruses, artificial chromosomes, or any nucleic acid vehicle known in the art suitable for amplification, transfer, and/or expression of a polynucleotide contained therein.

As used herein, unless as otherwise described with regard to viral vectors, "vector" means a nucleic acid molecule capable of transporting another nucleic acid to which it has been linked. Exemplary vectors include plasmids, minicircles, transposons (e.g., Sleeping Beauty transposon), yeast artificial chromosomes, self-replicating RNAs, and viral genomes. Certain vectors can autonomously replicate in a host cell, while other vectors can be integrated into the genome of a host cell and thereby are replicated with the host genome. In addition, certain vectors are referred to herein as "recombinant expression vectors" (or simply, "expression vectors"), which contain nucleic acid sequences that are operatively linked to an expression control sequence and, therefore, are capable of directing the expression of those sequences. In certain embodiments, expression constructs are derived from plasmid vectors. Illustrative constructs include modified pNASS vector (Clontech, Palo Alto, CA), which has nucleic acid sequences encoding an ampicillin resistance gene, a polyadenylation signal and a T7 promoter site; pDEF38 and pNEF38 (CMC ICOS Biologies, Inc.), which have a CHEF1 promoter; and pD18 (Lonza), which has a CMV promoter. Other suitable mammalian expression vectors are well known (see, *e.g*., Ausubel et al., 1995; Sambrook et al., supra; see also, e.g., catalogs from Invitrogen, San Diego, CA; Novagen, Madison, W1; Pharmacia, Piscataway, NJ).

Useful constructs may be prepared that include a dihydrofolate reductase (DHFR)-encoding sequence under suitable regulatory control, for promoting enhanced production levels of the fusion proteins, which levels result from gene amplification following application of an appropriate selection agent (*e.g.*, methotrexate). In one embodiment, use of a bifunctional transposon encoding a therapeutic gene (e.g., FST) along with drug-resistant DHFR in combination with incubation in methotrexate (MTX) to enrich for successfully transposed B cells, generates a more potent product.

Generally, recombinant expression vectors will include origins of replication and selectable markers permitting transformation of the host cell, and a promoter derived from a highly-expressed gene to direct transcription of a downstream structural sequence, as described above. A vector in operable linkage with a polynucleotide according to this disclosure yields a cloning or expression construct. Exemplary cloning/expression constructs contain at least one expression control element, *e.g*., a promoter, operably linked to a polynucleotide of this disclosure. Additional expression control elements, such as enhancers, factor-specific binding sites, terminators, and ribosome binding sites are also contemplated in the vectors and cloning/expression constructs according to this disclosure. The heterologous structural sequence of the polynucleotide according to this disclosure is assembled in appropriate phase with translation initiation and termination sequences. Thus, for example, encoding nucleic acids as provided herein may be included in any one of a variety of expression vector constructs (*e.g*., minicircles) as a recombinant expression construct for expressing such a protein in a host cell.

The appropriate DNA sequence(s) may be inserted into a vector, for example, by a variety of procedures. In general, a DNA sequence is inserted into an appropriate restriction endonuclease cleavage site(s) by procedures known in the art. Standard techniques for cloning, DNA isolation, amplification and purification, for enzymatic reactions involving DNA ligase, DNA polymerase, restriction endonucleases and the like, and various separation techniques are contemplated. A number of standard techniques are described, for example, in Ausubel et al. (Current Protocols in Molecular Biology, Greene Publ. Assoc. Inc. & John Wiley & Sons, Inc., Boston, MA, 1993); Sambrook et al. (Molecular Cloning, Second Ed., Cold Spring Harbor Laboratory, Plainview, NY, 1989); Maniatis et al. (Molecular Cloning, Cold Spring Harbor Laboratory, Plainview, NY, 1982); Glover (Ed.) (DNA Cloning Vol. I and II, IRL Press, Oxford, UK, 1985); Hames and Higgins (Eds.) (Nucleic Acid Hybridization, IRL Press, Oxford, UK, 1985); and elsewhere.

The DNA sequence in the expression vector is operatively linked to at least one appropriate expression control sequence (*e.g*., a constitutive promoter or a regulated promoter) to direct mRNA synthesis. Representative examples of such expression control sequences include promoters of eukaryotic cells or their viruses, as described above. Promoter regions can be selected from any desired gene using CAT (chloramphenicol transferase) vectors, kanamycin vectors, or other vectors with selectable markers. Eukaryotic promoters include CMV immediate early, HSV thymidine kinase, early and late SV40, LTRs from retrovirus, and mouse metallothionein-1. Selection of the appropriate vector and promoter is well within the level of ordinary skill in the art, and preparation of certain particularly preferred recombinant expression constructs comprising at least one promoter or regulated promoter operably linked to a nucleic acid encoding a protein or polypeptide according to this disclosure is described herein.

Variants of the polynucleotides of this disclosure are also contemplated. Variant polynucleotides are at least 60%, 65%, 70%, 75%, 80%, 85%, 90%, and preferably 95%, 96%, 97%, 98%, 99%, or 99.9% identical to one of the polynucleotides of defined sequence as described herein, or that hybridizes to one of those polynucleotides of defined sequence under stringent hybridization conditions of 0.015M sodium chloride, 0.0015M sodium citrate at about 65-68°C or 0.015M sodium chloride, 0.0015M sodium citrate, and 50% formamide at about 42°C. The polynucleotide variants retain the capacity to encode a binding domain or fusion protein thereof having the functionality described herein.

The term "stringent" is used to refer to conditions that are commonly understood in the art as stringent. Hybridization stringency is principally determined by temperature, ionic strength, and the concentration of denaturing agents such as formamide. Examples of stringent conditions for hybridization and washing are 0.015M sodium chloride, 0.0015M sodium citrate at about 65-68°C or 0.015M sodium chloride, 0.0015M sodium citrate, and 50% formamide at about 42°C (see Sambrook et ai, Molecular Cloning: A Laboratory Manual, 2nd Ed., Cold Spring Harbor Laboratory, Cold Spring Harbor, N.Y., 1989). More stringent conditions (such as higher temperature, lower ionic strength, higher formamide, or other denaturing agent) may also be used; however, the rate of hybridization will be affected. In instances wherein hybridization of deoxyoligonucleotides is concerned, additional exemplary stringent hybridization conditions include washing in 6x SSC, 0.05% sodium pyrophosphate at 37°C (for 14-base oligonucleotides), 48°C (for 17-base oligonucleotides), 55°C (for 20-base oligonucleotides), and 60°C (for 23-base oligonucleotides).

A further aspect of this disclosure relates to a host cell transformed or transfected with, or otherwise containing, any of the polynucleotides or vector/expression constructs of this disclosure (e.g., a follistatin polynucleotides or vector/expression constructs). The polynucleotides or cloning/expression constructs of this disclosure are introduced into suitable cells using any method known in the art, including transformation, transfection and transduction (e.g., any one of the methods disclosed herein). Host cells include the cells of a subject undergoing *ex vivo* cell therapy including, for example, *ex vivo* gene therapy. Eukaryotic host cells contemplated as an aspect of this disclosure when harboring a polynucleotide, vector, or protein according to this disclosure include, in addition to a subject's own cells (*e.g*., a human patient's own cells), VERO cells, HeLa cells, Chinese hamster ovary (CHO) cell lines (including modified CHO cells capable of modifying the glycosylation pattern of expressed multivalent binding molecules, see US Patent Application Publication No. 2003/01 15614), COS cells (such as COS-7), W138, BHK, HepG2, 3T3, RIN, MDCK, A549, PC12, K562, HEK293 cells, HepG2 cells, N cells, 3T3 cells, Spodoptera frugiperda cells (*e.g*., Sf9 cells), Saccharomyces cerevisiae cells, and any other eukaryotic cell known in the art to be useful in expressing, and optionally isolating, a protein or peptide according to this disclosure. Also contemplated are prokaryotic cells, including Escherichia coli, Bacillus subtilis, Salmonella typhimurium, a Streptomycete, or any prokaryotic cell known in the art to be suitable for expressing, and optionally isolating, a protein or peptide according to this disclosure. In isolating protein or peptide from prokaryotic cells, in particular, it is contemplated that techniques known in the art for extracting protein from inclusion bodies may be used. The selection of an appropriate host is within the scope of those skilled in the art from the teachings herein. Host cells that glycosylate the fusion proteins of this disclosure are contemplated.

The term "recombinant host cell" (or simply "host cell") refers to a cell containing a recombinant expression vector. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. Recombinant host cells can be cultured in a conventional nutrient medium modified as appropriate for activating promoters, selecting transformants, or amplifying particular genes. The culture conditions for particular host cells selected for expression, such as temperature, pH and the like, will be readily apparent to the ordinarily skilled artisan. Various mammalian cell culture systems can also be employed to express recombinant protein. Examples of mammalian expression systems include the COS-7 lines of monkey kidney fibroblasts, described by Gluzman (1981) Cell 23:175, and other cell lines capable of expressing a compatible vector, for example, the C127, 3T3, CHO, HeLa and BHK cell lines. Mammalian expression vectors will comprise an origin of replication, a suitable promoter and, optionally, enhancer, and also any necessary ribosome binding sites, polyadenylation site, splice donor and acceptor sites, transcriptional termination sequences, and 5'-flanking nontranscribed sequences, for example, as described herein regarding the preparation of multivalent binding protein expression constructs. DNA sequences derived from the SV40 splice, and polyadenylation sites may be used to provide the required nontranscribed genetic elements. Introduction of the construct into the host cell can be effected by a variety of methods with which those skilled in the art will be familiar, including calcium phosphate transfection, DEAE-Dextran-mediated transfection, or electroporation (Davis et al. (1986) Basic Methods in Molecular Biology).

### Cells and Compositions

In one embodiment, the modified B cells described herein have been activated/differentiated *in vitro* and transfected to express a therapeutic agent as described herein (e.g., follistatin). In one embodiment, the modified B cells described herein have been activated/differentiated *in vitro* and engineered (e.g., using a targeted transgene integration approach such as a zinc finger nuclease, TALEN, meganuclease, or CRISPR/CAS9-mediated transgene integration) to express a therapeutic agent as described herein (e.g., follistatin). In one embodiment, the compositions comprise B cells that have differentiated into plasma B cells, have been transfected or otherwise engineered and express one or more proteins of interest (e.g., follistatin). Target cell populations, such as the transfected or otherwise engineered and activated B cell populations of the present disclosure may be administered either alone, or as a pharmaceutical composition in combination with diluents and/or with other components such as cytokines or cell populations.

In one embodiment, the modified B cells that have been engineered to express one or more proteins of interest (e.g., follistatin) are harvested from culture after activation/differentiation *in vitro* at a time-point at which the modified B cells have optimal migratory capacity for a particular chemoattractant. In some embodiments, the optimal migratory capacity may be on day 7, day 8, or day 9 of the B cell culture. In some embodiments, the optimal migratory capacity may be on day 5, day 6, or day 7 of the B cell culture after transfection or engineering. In some embodiments, the optimal migratory capacity may be on day 8 of the B cell culture after transfection or engineering or later in culture than day 8 after transfection or engineering (e.g., day 9, 10, 11, 12, 13, 14, 15, 16, 17, 18, 19, 20, or later than day 20). In some embodiments, the optimal migratory capacity may be prior to day 10 of the B cell culture. In some embodiments, the optimal migratory capacity may be prior to day 8 of the B cell culture after transfection or engineering. In some embodiments, the optimal migratory capacity may be on day 6 or day 7 of the B cell culture. In some embodiments, the optimal migratory capacity may be on day 4 or day 5 of the B cell culture after transfection or engineering. In some embodiments, the optimal migratory capacity may be prior to day 9 of the B cell culture. In some embodiments, the optimal migratory capacity may be prior to day 7 of the B cell culture after transfection or engineering. In some embodiments, the optimal migratory capacity is optimal for modified B cell homing to CXCL12. In some embodiments, the optimal migratory capacity is optimal for modified B cell homing to the bone marrow of a subject receiving one or more administration of the modified B cells. In some embodiments, the B cells are harvested for administration to a subject at optimal migratory capacity to CXCL12 and/or to the bone marrow of a subject on from about day 7 to about day 9 in culture. In some embodiments, the B cells are harvested for administration to a subject at optimal migratory capacity to CXCL12 and/or to the bone marrow of a subject on from about day 5 to about day 7 in culture after transfection or engineering. In some embodiments, the B cells are harvested for administration to a subject at optimal migratory capacity to CXCL12 and/or to the bone marrow of a subject prior to about day 10 in culture. In some embodiments, the B cells are harvested for administration to a subject at optimal migratory capacity to CXCL12 and/or to the bone marrow of a subject prior to about day 8 in culture after transfection or engineering. In some embodiments, the optimal migratory capacity is optimal for modified B cell homing to CXCL13. In some embodiments, the optimal migratory capacity is optimal for modified B cell homing to a site of inflammation in a subject receiving one or more administration of the modified B cells. In some embodiments, the B cells are harvested for administration to a subject at optimal migratory capacity to CXCL13 and/or to a site of inflammation in the subject on about day 6 or about day 7 in culture. In some embodiments, the B cells are harvested for administration to a subject at optimal migratory capacity to CXCL 13 and/or to a site of inflammation in the subject on about day 4 or about day 5 in culture after transfection or engineering. In some embodiments, the B cells are harvested for administration to a subject at optimal migratory capacity to CXCL13 and/or to a site of inflammation prior to about day 10 in culture. In some embodiments, the B cells are harvested for administration to a subject at optimal migratory capacity to CXCL13 and/or to a site of inflammation prior to about day 8 in culture after transfection or engineering.

In some embodiments, the optimal migratory capacity is optimal for modified B cell homing to both CXCL12 and CXCL13. In some embodiments, the B cells are harvested at optimal migratory capacity for homing to both CXCL12 and CXCL13 on day 7 of the B cell culture. In some embodiments, the B cells are harvested at optimal migratory capacity for homing to both CXCL12 and CXCL13 on day 5 of the B cell culture after transfection or engineering.

In some embodiments, the engineered B cells are harvested when at least about 20%, of the B cells migrate in a chemotaxis assay to a particular chemoattractant. For example, but not to be limited by example, the engineered B cells (e.g., that produce FST) may be harvested when at least about 20% of the B cells migrate in a chemotaxis assay to CXCL12. Or, in another non-limiting example, the engineered B cells (e.g., that produce FST) may be harvested when at least about 20% of the B cells migrate in a chemotaxis assay to CXCL13. Furthermore, the engineered B cells (e.g., that produce FST) may be harvested when at least about 30% of the B cells migrate in a chemotaxis assay to a particular chemoattractant (e.g., CXCL12 or CXCL13), or when at least about 40%, 45%, 50%, 55%, 60%, 65%, or at least about 70% of the B cells migrate in a chemotaxis assay to a particular chemoattractant (e.g., CXCL12 or CXCL13). Furthermore, the engineered B cells (e.g., that produce FST) may be harvested when more than 70% of the B cells migrate in a chemotaxis assay. Such chemotaxis assays are known in the art.

Briefly, cell compositions of the present disclosure may comprise a differentiated and activated B cell population that has been transfected and is expressing a therapeutic agent as described herein (e.g., follistatin), in combination with one or more pharmaceutically or physiologically acceptable carriers, diluents or excipients. Such compositions may comprise buffers such as neutral buffered saline, phosphate buffered saline, Lactated Ringer's solution and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are preferably formulated for intravenous or subcutaneous administration.

In one embodiment, a cell composition is assessed for purity prior to administration. In another embodiment, a cell composition is tested for robustness of therapeutic agent production. In one embodiment, a cell composition is tested for sterility. In another embodiment, a cell composition is screened to confirm it matches the recipient subject.

In one embodiment, an engineered B cell population is assessed for polyclonality prior to administration to a subject. In some embodiments, ensuring polyclonality of the final cell product is an important safety parameter. Specifically, the emergence of a dominant clone may be viewed as potentially contributing to *in vivo* tumorigenesis or auto-immune disease. Polyclonality may be assessed by any means known in the art or described herein. For example, in some embodiments, polyclonality is assessed by sequencing (e.g., by deep sequencing) the B cell receptors expressed in an engineered B cell population. Since the B cell receptor undergoes changes during B cell development that makes it unique between B cells, this method allows for quantifying how many cells share the same B cell receptor sequence (meaning they are clonal). Thus, in some embodiments, the more B cells in an engineered B cell population that express the same B cell receptor sequence, the more clonal the population and, therefore, the less safe the population is for administration to a subject. Conversely, in some embodiments, the less B cells in an engineered B cell population that express the same B cell receptor sequence, the less clonal the population *(i.e.,* more polyclonal) and, thus, the more safe the population is for administration to a subject.

In some embodiments, the engineered B cells are administered to a subject after they have been determined to be sufficiently polyclonal. For example, the engineered B cells may be administered to a subject after it has been determined that no particular B cell clone in the final population comprises more than about 0.2% of the total B cell population. The engineered B cells may be administered to a subject after it has been determined that no particular B cell clone in the final population comprises more than about 0.1% of the total B cell population, or more than about 0.09%, 0.08%, 0.07%, 0.06%, 0.05%, or about 0.04%, of the total B cell population. In particular embodiments, the engineered B cells (e.g., which produce (follistatin)) are administered to a subject after it has been determined that no particular B cell clone in the final population comprises more than about 0.03% of the total B cell population.

In one embodiment, a cell composition is stored and/or shipped at 4°C. In another embodiment, a cell composition is frozen for storage and/or shipment. A cell composition may be frozen at, *e.g*., -20°C or -80°C. In one embodiment, a step of freezing a cell composition comprises liquid nitrogen. In one embodiment, a cell composition is frozen using a controlled rate freezer. Accordingly, methods described herein may further include a thawing step.

### Methods of Use

One aspect of the present invention is directed to the *in vivo* delivery of a therapeutic agent (e.g., follistatin) via delivery of a modified B cell engineered to express the therapeutic agent (e.g., follistatin). In particular embodiments, B cells modified to express follistatin are used in methods of treating and/or preventing chronic diseases and disorders and/or in methods of increasing muscle size or strength in a patient..

Modified B cells described herein may be administered in a manner appropriate to the disease or disorder to be treated or prevented.

The quantity and frequency of administration will be determined by such factors as the condition of the patient, and the type and severity of the patient's disease, although appropriate dosages may be determined by clinical trials.

In one embodiment, a single dose of modified B cells is administered to a subject. In one embodiment, two or more doses of modified B cells are administered sequentially to a subject. In one embodiment, three doses of modified B cells are administered sequentially to a subject. In one embodiment, a dose of modified B cells is administered weekly, biweekly, monthly, bimonthly, quarterly, semiannually, annually, or biannually to a subject. In one embodiment, a second or subsequent dose of modified B cells is administered to a subject when an amount of a therapeutic agent produced by the modified B cells decreases.

In one embodiment, a dose of modified B cells is administered to a subject at a certain frequency (e.g., weekly, biweekly, monthly, biomonthly, or quarterly) until a desired amount (e.g., an effective amount) of a therapeutic agent (e.g., follistatin) is detected in the subject. In one embodiment, an amount of the therapeutic agent (e.g., follistatin) is monitored in the subject. In one embodiment, a subsequent dose of modified B cells is administered to the subject when the amount of the therapeutic agent produced by the modified B cells decreases below the desired amount. In one embodiment, the desired amount is a range that produces the desired effect. For example, in a method for treating a muscular dystrophy (e.g., Becker Muscular Dystrophy), a desired amount of follistatin may be an amount in the plasma of a subject receiving the modified B cells. In some embodiments, the desired amount may be an amount of follistatin that results in a certain level of weight gain by the subject. In some embodiments, the desired amount may be an amount of follistatin that results in a certain level of increased strength by the subject. In some embodiments, the desired amount may be an amount of follistatin that results in a certain level of increased body mass of the subject.

When "an effective amount" or "therapeutic amount" is indicated, the precise amount of the compositions of the present disclosure to be administered can be determined by a physician with consideration of individual differences in age, weight, tumor size, extent of infection or metastasis, and condition of the patient (subject). B cell compositions may also be administered multiple times at an appropriate dosage(s). The cells can be administered by using infusion techniques that are commonly known in immunotherapy (see, e.g., Rosenberg et al., New Eng. J. of Med. 319:1676, 1988).

The optimal dosage and treatment regime for a particular patient can be determined by one skilled in the art of medicine by monitoring the patient for signs of disease and adjusting the treatment accordingly. The treatment may also be adjusted after measuring the levels of a therapeutic agent (e.g., follistatin) in a biological sample (e.g., body fluid such as plasma or a tissue sample) can also be used to assess the treatment efficacy, and the treatment may be adjusted accordingly to increase or decrease.

In some aspects of the present disclosure, an optimal dosage of the modified B cells for a multi-dose regime may be determined by first determining an optimal single-dose concentration of the B cells for a subject, decreasing the number of B cells present in the optimal single-dose concentration to provide a sub-optimal single-dose concentration of the modified B cells, and administering two or more dosages of the sub-optimal single-dose concentration of modified B cells to the subject. In some aspects, 2, 3, or more dosages of a sub-optimal single-dose concentration of modified B cells are administered to the subject. In some aspects, the administration of 2, 3, or more dosages of a sub-optimal single-dose concentration of modified B cells to a subject results in synergistic in vivo production of a therapeutic polypeptide that the modified B cells are engineered to express. In some aspects, the sub-optimal single-dose concentration comprises 1/2 or 3, 4, 5, 6, 7, 8, 9, 10 fold, or less than the optimal single-dose concentration. In some aspects, the therapeutic polypeptide is follistatin.

In some aspects of the present disclosure, lower numbers of the transfected B cells of the present disclosure, in the range of 106/kilogram (106-1011 per patient) may be administered. In certain embodiments, the B cells are administered at 1 x 104, 5 x 104, 1 x 105, 5 x 105, 1 x 106, 5 x 106, 1 x 107, 5 x 107, 1 x 108, 5 x 108, 5 x 109, 1 x 1010, 5 x 1010, 1 x 1011, 5 x 1011, or 1 x 1012 cells to the subject. B cell compositions may be administered multiple times at dosages within these ranges. The cells may be autologous or heterologous (e.g., allogeneic) to the patient undergoing therapy. If desired, the treatment may also include administration of mitogens (e.g., PHA) or lymphokines, cytokines, and/or chemokines (e.g., GM-CSF, IL-4, IL-6, IL-13, IL-21, Flt3-L, RANTES, MIP1α, BAFF, etc.) as described herein to enhance induction of an immune response and engraftment of the infused B cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intrathecally, intramuscularly, by intravenous (i.v.) injection, or intraperitoneally. The compositions described herein may be administered to a patient directly into the nervous system. In one embodiment, the B cell compositions of the present disclosure are administered to a patient by intradermal or subcutaneous injection. In another embodiment, the B cell compositions as described herein are preferably administered by i.v. injection. The compositions of B cells may be injected directly into a tumor, lymph node, bone marrow or site of infection.

In yet another embodiment, the pharmaceutical composition can be delivered in a controlled release system. In one embodiment, a pump may be used (see Langer, 1990, Science 249:1527-1533; Sefton 1987, CRC Crit. Ref. Biomed. Eng. 14:201; Buchwald et al., 1980; Surgery 88:507; Saudek et al., 1989, N. Engl. J. Med. 321 :574). In another embodiment, polymeric materials can be used (see Medical Applications of Controlled Release, 1974, Langer and Wise (eds.), CRC Pres., Boca Raton, Fla.; Controlled Drug Bioavailability, Drug Product Design and Performance, 1984, Smolen and Ball (eds.), Wiley, New York; Ranger and Peppas, 1983; J. Macromol. Sci. Rev. Macromol. Chem. 23:61 ; see also Levy et al., 1985, Science 228:190; During et al., 1989, Ann. Neurol. 25:351 ; Howard et al., 1989, J. Neurosurg. 71 :105). In yet another embodiment, a controlled release system can be placed in proximity of the therapeutic target, thus requiring only a fraction of the systemic dose (see, e.g., Medical Applications of Controlled Release, 1984, Langer and Wise (eds.), CRC Pres., Boca Raton, Fla., vol. 2, pp. 1 15-138).

The B cell compositions of the present disclosure may also be administered using any number of matrices. Matrices have been utilized for a number of years within the context of tissue engineering (see, e.g., Principles of Tissue Engineering (Lanza, Langer, and Chick (eds.)), 1997. The present disclosure utilizes such matrices within the novel context of acting as an artificial lymphoid organ to support and maintain the B cells. Accordingly, the present disclosure can utilize those matrix compositions and formulations which have demonstrated utility in tissue engineering. Accordingly, the type of matrix that may be used in the compositions, devices and methods of the disclosure is virtually limitless and may include both biological and synthetic matrices. In one particular example, the compositions and devices set forth by U.S. Patent Nos: 5,980,889; 5,913,998; 5,902,745; 5,843,069; 5,787,900; or 5,626,561 are utilized. Matrices comprise features commonly associated with being biocompatible when administered to a mammalian host. Matrices may be formed from natural and/or synthetic materials. The matrices may be nonbiodegradable in instances where it is desirable to leave permanent structures or removable structures in the body of an animal, such as an implant; or biodegradable. The matrices may take the form of sponges, implants, tubes, telfa pads, fibers, hollow fibers, lyophilized components, gels, powders, porous compositions, or nanoparticles. In addition, matrices can be designed to allow for sustained release seeded cells or produced cytokine or other active agent. In certain embodiments, the matrix of the present disclosure is flexible and elastic, and may be described as a semisolid scaffold that is permeable to substances such as inorganic salts, aqueous fluids and dissolved gaseous agents including oxygen.

A matrix is used herein as an example of a biocompatible substance. However, the current disclosure is not limited to matrices and thus, wherever the term matrix or matrices appears these terms should be read to include devices and other substances which allow for cellular retention or cellular traversal, are biocompatible, and are capable of allowing traversal of macromolecules either directly through the substance such that the substance itself is a semi-permeable membrane or used in conjunction with a particular semi-permeable substance.

In certain embodiments of the present disclosure, B cells transfected and activated using the methods described herein, or other methods known in the art, are administered to a patient in conjunction with (e.g. before, simultaneously or following) any number of relevant treatment modalities, including but not limited to treatment with agents such as antiviral agents, chemotherapy, radiation, immunosuppressive agents, such as cyclosporin, bisulfin, bortezomib, azathioprine, methotrexate, mycophenolate, and FK506, antibodies, or other immunoablative agents such as CAMPATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506), the proteasome (bortezomib), or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993; Isoniemi (supra)). In a further embodiment, the cell compositions of the present disclosure are administered to a patient in conjunction with (e.g. before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In one embodiment, the cell compositions of the present disclosure are administered following B-cell ablative therapy such as agents that react with CD20, e.g. Rituxan^{®}. In one embodiment, the cell compositions of the present disclosure are administered following B cell ablative therapy using an agent such as bortezomib. For example, in one embodiment, subjects may undergo standard treatment with high dose chemotherapy followed by peripheral blood stem cell transplantation. In certain embodiments, following the transplant, subjects receive an infusion of the expanded immune cells of the present disclosure. In an additional embodiment, expanded cells are administered before or following surgery.

The dosage of the above treatments to be administered to a patient will vary with the precise nature of the condition being treated and the recipient of the treatment. The scaling of dosages for human administration can be performed according to art-accepted practices.

The modified B cells can be used in the treatment or prevention of various diseases and disorders. In particular embodiments, B cells modified to express follistatin are used in methods of increasing muscle size or strength in a patient. For example, the disclosure relates to a method of increasing muscle size or strength in a subject, the method comprising administering an effective amount of a B cell expressing a follistatin polypeptide. The increased muscle size or strength may occur generally throughout the subject or it may occurs in a targeted muscle. The targeted muscle may be damaged, weakened or deficient, as may be the case in a variety of muscle disorders including muscular dystrophies (such as Duchenne muscular dystrophy, Becker's muscular dystrophy, Emery Dreifuss muscular dystrophy, limb girdle muscular dystrophy, rigid spine syndrome, Ulirich syndrome, Fukuyama muscular dystrophy, Walker-Warburg syndrome, muscle-eye-brain disease, fascioscapulohumeral muscular dystrophy), congenital muscular dystrophy, myotonic dystrophy (Steinert's disease), nondystrophic myotonia, periodic paralyses spinal muscular atrophy, familial amytrophic lateral sclerosis, hereditary motor and sensory neuropathy, Charcot-Marie-Tooth disease, chronic inflammatory neuropathy, distal myopathy, myotubular/centronuclear myopathy, nemaline myopathy, mini core disease, central core disease, desminopathy, inclusion body myositis, mitochondrial myopathy, congenital myasthenic syndrome, post-polio muscle dysfunction, and disorders described in Emery (2002) The Lancet, 359:687-695; and Khurana et al. (2003) Nat. Rev. Drug Disc., 2:379-386), inflammatory muscle disorders (such as inclusion body myositis), muscle injury or trauma, muscle disuse (as may occur after prolonged bed rest or limb immobilization) and muscle atrophy or weakening as a consequence of aging, cancer or chronic diseases of various types. For example, in some instances, the muscle may be damaged, weakened, or deficient due to sarcopenia. In some instances, the muscle may be damaged, weakened, or deficient due to spinal muscular atrophy (SMA). In some instances, the muscle may be damaged, weakened, or deficient due to amyotrophic lateral sclerosis (ALS). In some instances, the muscle may be damaged, weakened, or deficient due to Pompe disease. The methods may also be increase the muscle size or strength in a muscle that is healthy.

In some embodiments the present disclosure relates to a method for treating a disease or disorder in an individual comprising administering a B cell genetically modified to express follistatin (e.g., follistatin+ B cells expressing the FST344 transcript variant) to a subject in need thereof.

In some embodiments, the present disclosure relates to a method for treating a muscle disorder in an individual comprising administering a B cell genetically modified to express follistatin (e.g., follistatin+ B cells expressing the FST344 transcript variant) to a subject having, or suspected of having such a muscle disorder, wherein the muscle disorder is a muscular dystrophy. In some embodiments, the muscular dystrophy is selected from Duchenne muscular dystrophy, Becker's muscular dystrophy, Emery Dreifuss muscular dystrophy, limb girdle muscular dystrophy, rigid spine syndrome, Ulirich syndrome, Fukuyama muscular dystrophy, Walker-Warburg syndrome, muscle-eye-brain disease, fascioscapulohumeral muscular dystrophy), congenital muscular dystrophy, myotonic dystrophy (Steinert's disease), nondystrophic myotonia, periodic paralyses spinal muscular atrophy, familial amytrophic lateral sclerosis, hereditary motor and sensory neuropathy, Charcot-Marie-Tooth disease, chronic inflammatory neuropathy, distal myopathy, myotubular/centronuclear myopathy, nemaline myopathy, mini core disease, central core disease, desminopathy, inclusion body myositis, mitochondrial myopathy, congenital myasthenic syndrome, post-polio muscle dysfunction, and disorders described in Emery (2002) The Lancet, 359:687-695; and Khurana et al. (2003) Nat. Rev. Drug Disc., 2:379-386).

In some embodiments, the present disclosure relates to a method for treating a muscle disorder in an individual comprising administering a B cell genetically modified to express follistatin (e.g., follistatin+ B cells expressing the FST344 transcript variant) to a subject having, or suspected of having such a muscle disorder, wherein the muscle disorder is an inflammatory muscle disorder. In some embodiments, the inflammatory disorder is inclusion body myositis.

In some embodiments, the present disclosure relates to a method for treating a muscle disorder in an individual comprising administering a B cell genetically modified to express follistatin (e.g., follistatin+ B cells expressing the FST344 transcript variant) to a subject having, or suspected of having such a muscle disorder, wherein the muscle disorder is caused by muscle injury or trauma.

In some embodiments, the present disclosure relates to a method for treating a muscle disorder in an individual comprising administering a B cell genetically modified to express follistatin (e.g., follistatin+ B cells expressing the FST344 transcript variant) to a subject having, or suspected of having such a muscle disorder, wherein the muscle disorder is caused by muscle disuse (e.g., as may occur after prolonged bed rest or limb immobilization).

In some embodiments, the present disclosure relates to a method for treating a muscle disorder in an individual comprising administering a B cell genetically modified to express follistatin (e.g., follistatin+ B cells expressing the FST344 transcript variant) to a subject having, or suspected of having such a muscle disorder, wherein the muscle disorder is selected from muscle atrophy or weakening as a consequence of aging, cancer or chronic diseases of various types.

In some embodiments, the present disclosure relates to a method for treating an individual exhibiting mild, moderate or severe muscle weakness, muscle wasting, and/or effects on independent ambulation comprising administering a B cell genetically modified to express follistatin (e.g., follistatin+ B cells expressing the FST344 transcript variant) the subject.

In some embodiments, the present disclosure relates to a method for treating an individual exhibiting mild, moderate or severe muscle fragility, muscle hypertrophy, muscle pseudohypertrophy, joint contracture, skeletal deformation, cardiomyopathy, impaired swallowing, impaired bowel and bladder function, muscle ischemia, cognitive impairment, behavioral dysfunction, socialization impairment, scoliosis, and/or impaired respiratory function comprising administering a B cell genetically modified to express follistatin (e.g., follistatin+ B cells expressing the FST344 transcript variant) the subject

In particular embodiments, the present disclosure relates to a method for treating a muscular dystrophy in an individual comprising administering a B cell genetically modified to express follistatin (e.g., follistatin+ B cells expressing the FST344 transcript variant) to a subject having, or suspected of having Becker Muscular Dystrophy.

In some embodiments, a single, maximally effective dose of follistatin+ B cells (e.g., follistatin+ B cells expressing the FST344 transcript variant) is administered to the subject. In some embodiments, two or more doses of follistatin+ B cells (e.g., follistatin+ B cells expressing the FST344 transcript variant) are administered to the subject, thereby maximizing the amount of engrafted follistatin + B cells. In some embodiments, the two or more doses of follistatin+ B cells (e.g., follistatin+ B cells expressing the FST344 transcript variant) that are administered to the subject comprise less follistatin + B cells than the single, maximally effective dose of follistatin + B cells. In some embodiments, when two or more doses of follistatin+ B cells (e.g., follistatin+ B cells expressing the FST344 transcript variant) are administered to a subject at a dosage of follistatin + B cells that is below the maximally effective single dose of follistatin+ B cells, a resultant synergistic increase in follistatin production occurs. In one embodiment, administering follistatin+ B cells to a subject results in normal levels of follistatin seen in a healthy, control subject. In one embodiment, administering follistatin+ B cells to a subject results in greater than normal levels of follistatin in the subject. In one embodiment, administering follistatin+ B cells (e.g., follistatin+ B cells expressing the FST344 transcript variant) to a subject increases the subject's strength. In one embodiment, administering follistatin+ B cells (e.g., follistatin+ B cells expressing the FST344 transcript variant) to a subject increases the subject's strength as compared to a normal level. In one embodiment, administering follistatin+ B cells (e.g., follistatin+ B cells expressing the FST344 transcript variant) to a subject prevents the subject from losing strength.

### EXAMPLES

### EXAMPLE 1

### PRODUCTION OF FOLLISTATIN EXPRESSING B CELLS

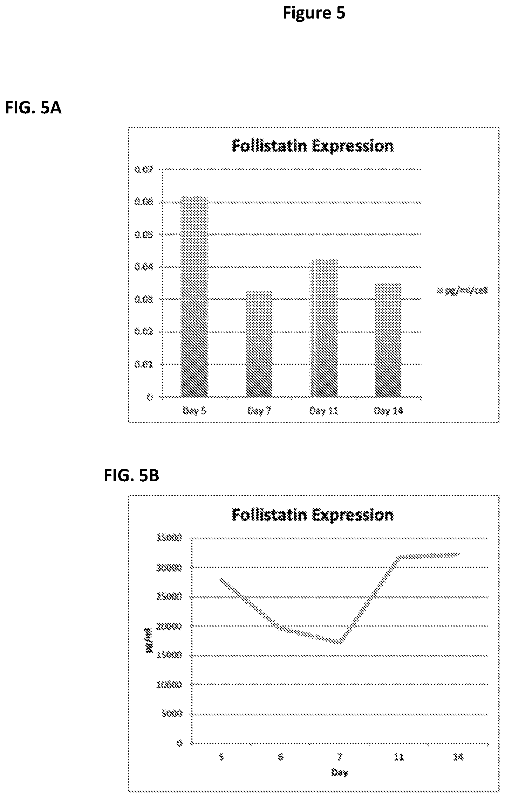

*Sleeping Beauty* transposon and transposase constructs for transposition and expression of human follistatin (FST) were generated. Transposons assembled to achieve FST gene integration and expression in B cells. We used the EEK promoter, consisting of promoter and enhancer elements from the human immunoglobulin gene as well as other regulatory elements previously described, to achieve high level expression in B cells. To test for FST transposition and expression, human B cells were isolated from two separate donors and expanded in culture in B cell culture medium and incubated at 37°C incubator with 5% CO2, electroporating on day 3 with pKT2/EEK-FST344 plus mRNA encoding SB100x transposase. Cell lysates prepared 2, 5, 8, and 11 days post-electroporation (*i.e*., days 5, 7, 11, and 14 in culture) contained significant increases in FST than wild-type non-transfected cells, demonstrating the effectiveness of the SB transposon system to achieve high-level FST expression in expanded human B cells (FIG. 5A). Notably, FST expression persisted from days 7-14, after a slight reduction from day 5 (likely due to initial episomal FST expression) suggesting stable integration of the construct into the B cells. Indeed RT-PCR confirmed stable integration of the FST insert into the B cell genome (FIG. 5B). These FST-expressing B cells are referred to as FST+B cells in the following examples.

### EXAMPLE 2

### IN VIVO PRODUCTION OF FST

To determine whether B cells engineered according to the present disclosure are able to facilitate *in vivo* increases in FST production, we injected wildtype mice with the FST+B cells produced in Example 1.

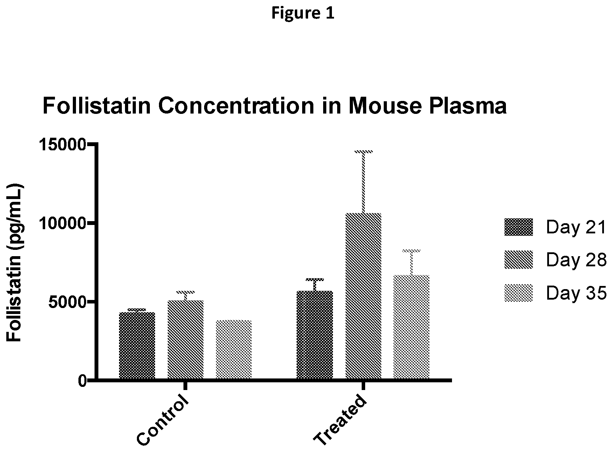

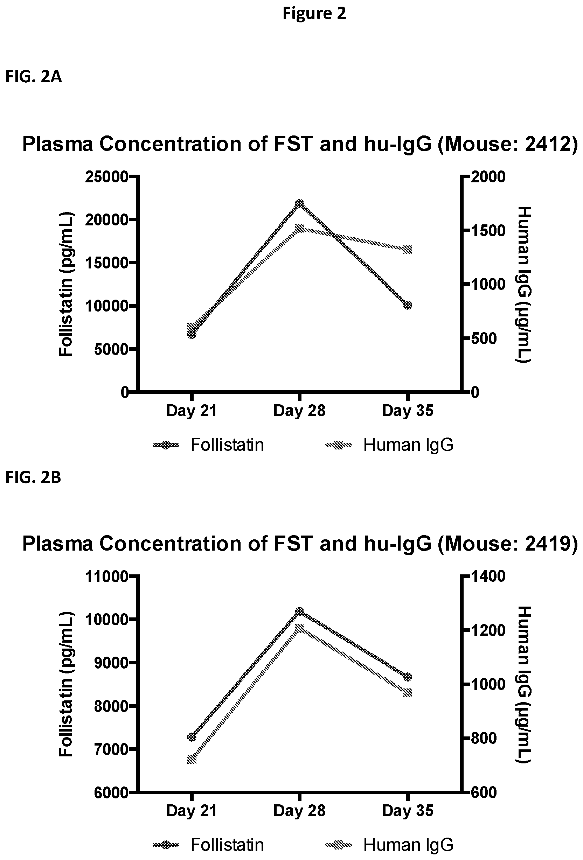

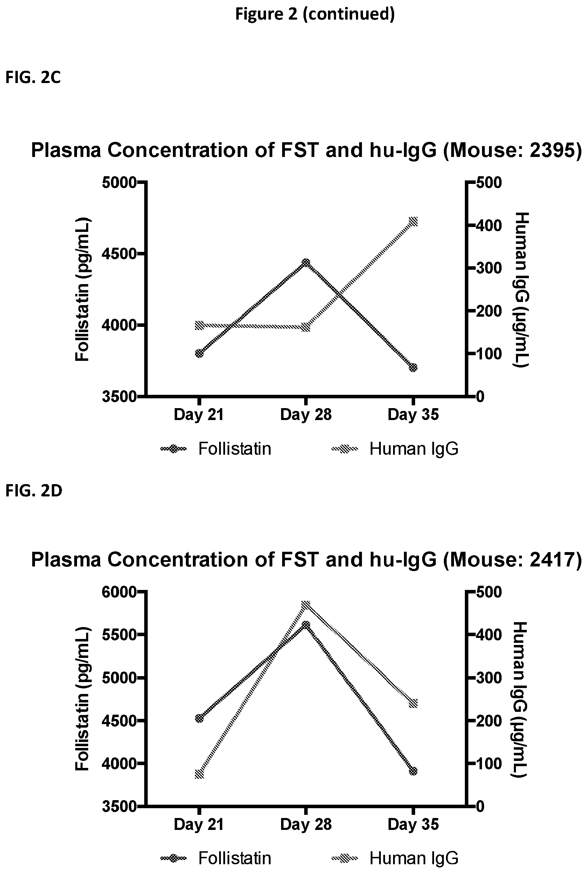

Specifically, four mice received an intravenous (tail vein) injections on day 0 of vehicle (500 µl phosphate buffered saline (PBS)) or 2 x 10⁶ human FST+B cells diluted in vehicle (PBS) to 500 µl. Additionally, mice were infused intraperitoneally (i.p.) on day -7 with 3x10⁶ primary autologous peripheral blood cells enriched for CD4+ T cells to provide support for the pKT2/EEK-FST plus mRNA encoding SB100x transposase transposed B cells. We observed a two-fold increase in human FST in plasma of mice treated with the FST+B cells providing strong evidence for successful human B cell adoptive transfer (Figure 1). FST levels peaked on round day 28 post-infusion, and had declined to near normal levels by Day 35. Although not carried out in FST deficient animals, the results from this experiment nonetheless provide an example of the levels of human FST that can be achieved after introduction of a highly potent FST+ B cell population wt mice. We found that plasma levels of FST correlated to plasma levels of human IgG; thus, providing evidence for adoptive transfer and engraftment of the FST+ B cells (Figure 2A-2D).

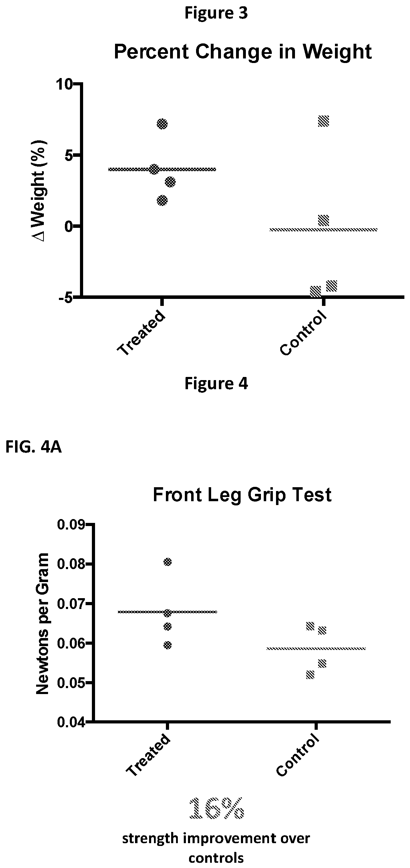

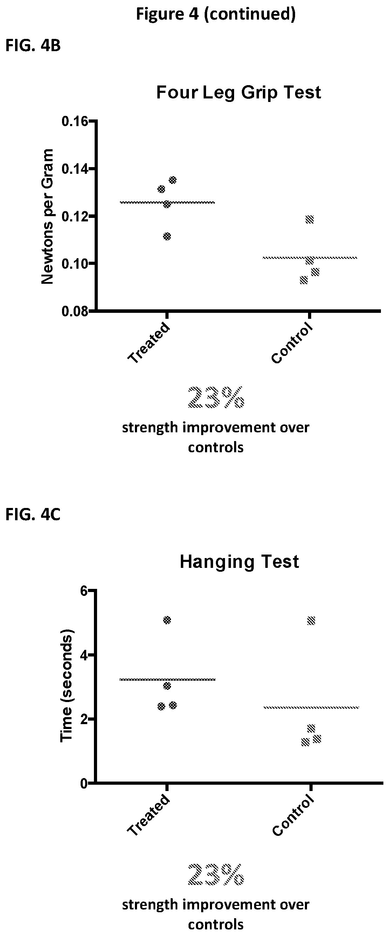

In order to determine whether the FST+B cells had any effect on treated mice, we monitored the weight of control and FST+B cell treated mice 35 days post-infusing. As shown in Figure 3, FST+B cells grew an average of 4.1% (0.9 grams) more than vehicle control-treated mice. Moreover, the increases in weight gain correspond to marked strength improvements in the front leg grip test (16% improvement in FST+B cell-treated mice as compared to vehicle controls)(FIG. 4A); four leg grip test (23% improvement in FST+B cell-treated mice as compared to vehicle controls)(FIG. 4B); and hanging test (23% improvement in FST+B cell-treated mice as compared to vehicle controls)(FIG. 4C).

Thus, these data demonstrate that B cells can be used in the methods disclosed herein to express and deliver FST to a subject to induce weight gain and improvements in strength.

The various embodiments described above can be combined to provide further embodiments. All of the U.S. patents, U.S. patent application publications, U.S. patent application, foreign patents, foreign patent application and non-patent publications referred to in this specification and/or listed in the Application Data Sheet relate to the description. The invention is defined by the appended claims.

## Claims

1. A recombinant B cell comprising a follistatin gene, wherein the B cell has been transduced or transposed with the follistatin gene operably linked to a promoter.

2. The B cell of claim 1, wherein the follistatin gene is a human follistatin gene, wherein
(i) the follistatin gene is a human follistatin FST-344 splice site variant,
(ii) the B cell is a human B cell, and/or
(iii) the follistatin gene encodes the amino acid sequence set forth in any one of SEQ ID NO: 1-4.

3. The B cell of claim 1 or 2, wherein:
(i) the B cell comprises the follistatin gene because it has been transduced with the follistatin gene using a transposon system, including wherein the transposon system is a sleeping beauty transposon system or a Piggybac transposon system; or
(ii) the B cell expresses the follistatin gene due to transduction with a virus carrying the follistatin gene, including wherein the B cell comprises the follistatin gene because it has been transduced with a retrovirus, lentivirus, adenovirus or adeno-associated virus comprising the follistatin gene; or
(iii) the B cell is engineered to contain the follistatin gene using a targeted integration approach, including wherein the targeted integration utilizes one or more zinc finger nucleases, transcription activator like effector nucleases (TALENs), and / or CRISPR/Cas systems including, but not limited to CRISPR/Cas9 systems; or
(iv) the B cell is engineered to contain the follistatin gene by introducing a follistatin-encoding nucleic acid using a method selected from the group consisting of retroviral vectors, lentiviral vectors, adeno-associated virus vectors, adenovirus vectors, any other RNA or DNA virus vectors, non-viral DNA and/or RNA encoding follistatin introduced using chemical or physical means such and lipofection, polycation complexation, electroporation, and the like.

4. The B cell of any one of the preceding claims, wherein the follistatin protein is secreted by the recombinant B cell.

5. The recombinant B cell of any one of claims 1-4, wherein the B cell is
(i) CD20-, CD38-, and CD138-,
(ii) CD20- and CD38+,
(iii) CD20-, CD38+, and CD138+, or
(iv) CD20-, CD38+, and CD138-.

6. The recombinant B cell of any one of claims 1-5, wherein the recombinant B cell is derived from
(i) a B cell obtained from a subject,
(ii) a B cell derived from a cell obtained from a subject
(iii) a B cell progenitor obtained from a subject, or
(iv) a cell obtained from the subject that has been dedifferentiated into the B cell or a B cell progenitor.

7. An in vitro method of producing recombinant B cells of any one of claims 1-6, wherein immune cells, such as CD19 positive cells, were collected and isolated from the blood of a subject; and engineered by
(i) transposing or transducing the cells with DNA encoding the follistatin, via electroporation such as utilizing the sleeping beauty transposon system;
(ii) expanding selected cells ex vivo; and
(iii) differentiating the expanded cells ex vivo into plasma cells and/or plasmablasts, wherein the differentiated cells are CD38(+) and CD20(-).

8. The method of claim 7, wherein the recombinant B cells are
(i) engineered on Day 2 or Day 3 after culturing,
(ii) harvested for administration to a subject on a day ranging from day 1 to day 12 of *in vitro* culture and on Day 4, Day 5, Day 6, Day 7 or Day 8 in culture after engineering, and/or
(iii) harvested at a time-point in culture at which it is determined that they do not produce significant levels of inflammatory cytokines, and/or
(iv) are grown in a culture system that comprises each of IL-2, IL-4, IL-10, IL-15, IL-21, and a multimerized CD40 ligand, a HIS tagged CD40 ligand that is multimerized using an anti-his antibody, throughout the entire culture period pre- and post-engineering.

9. The method of claim 7 or 8, further comprising expanding the genetically modified B cells prior to the administering to the subject, wherein:
(i) the final population of expanded genetically modified B cells demonstrates a high degree of polyclonality, and/or
(ii) any particular B cell clone in the final population of expanded genetically modified B cells comprises less than 0.05% or less than 0.2% of the total B cell population.

10. The method of any one of claims 7-9, wherein the genetically modified B cells comprise a polynucleotide encoding a selectable marker, wherein the selectable marker is a human DHFR gene with enhanced resistance to methotrexate, containing substitution mutations of leucine to tyrosine at amino acid 22 and phenylalanine to serine at amino acid 31.

11. The method of any one of claims 7-10, comprising treating the genetically modified B cells with methotrexate prior to harvesting for administration, wherein the methotrexate treatment is between 100 nM and 300 nM.

12. Recombinant B cells obtained by the method of any one of claims 7-11.

13. The recombinant B cells of any one of claims 1-5 or 12 for use in a method for treating, preventing or ameliorating a muscle disorder, wherein the muscle disorder is selected from the group consisting of a muscular dystrophy such as Duchenne muscular dystrophy, Becker's muscular dystrophy or fascioscapulohumeral muscular dystrophy, an inflammatory muscle disorder such as inclusion body myositis, muscle injury or trauma, muscle disuse such as after prolonged bed rest or limb immobilization, sarcopenia, spinal muscular atrophy (SMA), amyotrophic lateral sclerosis (ALS), Pompe disease, central core disease, and muscle atrophy or weakening such as after aging, cancer or chronic diseases.

14. The recombinant B cells for the use of claim 13, wherein the method comprises administering two or more sequential doses of genetically modified B cells to a subject, wherein
(i) administering comprises two, three or more doses of the genetically modified B cells at sub-optimal single-dose concentrations, and/or
(ii) the genetically modified B cells are autologous or allogenic to the subject, and/or
(iii) wherein the subject is human.

15. The recombinant B cells for the use of claim 13 or 14, wherein the B cells are formulated for intravenous, intraperitoneal, subcutaneous, intrathecal, or intramuscular injection.

## Patentansprüche

1. Eine rekombinante B-Zelle, die ein Follistatin-Gen umfasst, wobei die B-Zelle mit dem Follistatin-Gen, das funktionsfähig an einen Promotor gekoppelt ist, transduziert oder transponiert wurde.

2. Die B-Zelle nach Anspruch 1, wobei das Follistatin-Gen ein humanes Follistatin-Gen ist, wobei
(i) das Follistatin-Gen eine humane Follistatin-FST-344-Spleißstellenvariante ist,
(ii) die B-Zelle eine humane B-Zelle ist, und/oder
(iii) das Follistatin-Gen die in einer der SEQ ID NO: 1-4 angegebene Aminosäuresequenz kodiert.

3. Die B-Zelle nach Anspruch 1 oder 2, wobei:
(i) die B-Zelle das Follistatin-Gen umfasst, weil sie unter Verwendung eines Transposon-Systems mit dem Follistatin-Gen transduziert wurde, einschließlich der Fälle, in denen das Transposon-System ein Sleeping Beauty-Transposon-System oder ein Piggybac-Transposon-System ist; oder
(ii) die B-Zelle das Follistatin-Gen aufgrund einer Transduktion mit einem das Follistatin-Gen tragenden Virus exprimiert, einschließlich der Fälle, in denen die B-Zelle das Follistatin-Gen umfasst, weil sie mit einem das Follistatin-Gen umfassenden Retrovirus, Lentivirus, Adenovirus oder adeno-assoziierten Virus transduziert wurde; oder
(iii) die B-Zelle so gentechnisch verändert ist, dass sie das Follistatin-Gen enthält, unter Verwendung eines gezielten Integrationsansatzes, einschließlich solcher Fälle, in denen die gezielte Integration eine oder mehrere Zinkfinger-Nukleasen, Transkriptionsaktivator-ähnliche Effektor-Nukleasen (TALENs) und/oder CRISPR/Cas-Systeme, einschließlich, aber nicht beschränkt auf CRISPR/Cas9-Systeme, nutzt; oder
(iv) die B-Zelle so gentechnisch verändert ist, dass sie das Follistatin-Gen enthält, indem eine Follistatin-kodierende Nukleinsäure unter Verwendung eines Verfahrens eingeführt wird, das aus der Gruppe ausgewählt ist bestehend aus retroviralen Vektoren, lentiviralen Vektoren, adeno-assoziierten Virusvektoren, Adenovirus-Vektoren, beliebigen anderen RNA- oder DNA-Virusvektoren, nicht-viraler DNA und/oder RNA, die Follistatin kodiert, eingeführt unter Verwendung chemischer oder physikalischer Mittel wie Lipofektion, Polykation-Komplexierung, Elektroporation und dergleichen.

4. Die B-Zelle nach einem der vorstehenden Ansprüche, wobei das Follistatin-Protein von der rekombinanten B-Zelle sekretiert wird.

5. Die rekombinante B-Zelle nach einem der Ansprüche 1 bis 4, wobei die B-Zelle
(i) CD20-, CD38- und CD138- ist,
(ii) CD20- und CD38+ ist,
(iii) CD20-, CD38+ und CD138+ ist, oder
(iv) CD20-, CD38+ und CD138- ist.

6. Die rekombinante B-Zelle nach einem der Ansprüche 1 bis 5, wobei die rekombinante B-Zelle abgeleitet ist von
(i) einer B-Zelle, die von einem Subjekt gewonnen wurde,
(ii) einer B-Zelle, die von einer von einem Subjekt gewonnenen Zelle stammt,
(iii) einer B-Zell-Vorläuferzelle, die von einem Subjekt gewonnen wurde, oder
(iv) einer Zelle, die von dem Subjekt gewonnen wurde und in die B-Zelle oder eine B-Zell-Vorläuferzelle dedifferenziert wurde.

7. Ein In-vitro-Verfahren zur Herstellung rekombinanter B-Zellen gemäß einem der Ansprüche 1 bis 6, wobei Immunzellen, wie beispielsweise CD19-positive Zellen, aus dem Blut eines Subjekts entnommen und isoliert wurden; und gentechnisch verändert wurden durch
(i) Transposition oder Transduktion der Zellen mit DNA, die für Follistatin kodiert, mittels Elektroporation, beispielsweise unter Verwendung des Sleeping Beauty-Transposon-Systems;
(ii) Ex-vivo-Vermehrung ausgewählter Zellen; und
(iii) Ex-vivo-Differenzierung der vermehrten Zellen zu Plasmazellen und/oder Plasmablasten, wobei die differenzierten Zellen CD38(+) und CD20(-) sind.

8. Das Verfahren nach Anspruch 7, wobei die rekombinanten B-Zellen
(i) am 2. oder 3. Tag nach der Kultivierung gentechnisch verändert werden,
(ii) zur Verabreichung an ein Subjekt an einem Tag zwischen dem 1. und 12. Tag der In-vitro-Kultivierung sowie am 4., 5., 6., 7. oder 8. Tag der Kultivierung nach der gentechnischen Veränderung geerntet werden, und/oder
(iii) zu einem Zeitpunkt in der Kultur geerntet werden, zu dem festgestellt wird, dass sie keine signifikanten Mengen an inflammatorischen Zytokinen produzieren, und/oder
(iv) in einem Kultursystem gezüchtet werden, das während der gesamten Kulturperiode vor und nach der gentechnischen Veränderung jeweils IL-2, IL-4, IL-10, IL-15, IL-21 und einen multimerisierten CD40-Liganden, einen HIS-markierten CD40-Liganden, der unter Verwendung eines Anti-His-Antikörpers multimerisiert wird, umfasst.

9. Das Verfahren nach Anspruch 7 oder 8, das ferner das Expandieren der gentechnisch veränderten B-Zellen vor der Verabreichung an das Subjekt umfasst, wobei:
(i) die endgültige Population der expandierten gentechnisch veränderten B-Zellen einen hohen Grad an Polyklonalität aufweist, und/oder
(ii) jeder einzelne B-Zellklon in der endgültigen Population der expandierten gentechnisch veränderten B-Zellen weniger als 0,05 % oder weniger als 0,2 % der gesamten B-Zellpopulation ausmacht.

10. Das Verfahren nach einem der Ansprüche 7 bis 9, wobei die gentechnisch veränderten B-Zellen ein Polynukleotid umfassen, das für einen Selektionsmarker kodiert, wobei der Selektionsmarker ein humanes DHFR-Gen mit erhöhter Resistenz gegenüber Methotrexat ist, das Substitutionsmutationen von Leucin zu Tyrosin an Aminosäure 22 und von Phenylalanin zu Serin an Aminosäure 31 enthält.

11. Das Verfahren nach einem der Ansprüche 7 bis 10, umfassend die Behandlung der gentechnisch veränderten B-Zellen mit Methotrexat vor der Entnahme zur Verabreichung, wobei die Methotrexat-Behandlung in einem Bereich zwischen 100 nM und 300 nM erfolgt.

12. Rekombinante B-Zellen, erhalten durch das Verfahren nach einem der Ansprüche 7 bis 11.

13. Die rekombinanten B-Zellen nach einem der Ansprüche 1-5 oder 12 zur Verwendung in einem Verfahren zur Behandlung, Vorbeugung oder Linderung einer Muskelerkrankung, wobei die Muskelerkrankung ausgewählt ist aus der Gruppe bestehend aus einer Muskeldystrophie wie der Duchenne-Muskeldystrophie, Becker-Muskeldystrophie oder fascioscapulohumeraler Muskeldystrophie, einer entzündlichen Muskelerkrankung wie Inklusionskörper-Myositis, einer Muskelverletzung oder einem Muskeltrauma, einer Muskelinaktivität wie nach längerer Bettruhe oder Ruhigstellung einer Extremität, Sarkopenie, spinaler Muskelatrophie (SMA), amyotropher Lateralsklerose (ALS), Morbus Pompe, Central-Core-Krankheit sowie einer Muskelatrophie oder -schwächung wie nach Alterung, Krebs oder chronischen Erkrankungen.

14. Die rekombinanten B-Zellen zur Verwendung nach Anspruch 13, wobei das Verfahren die Verabreichung von zwei oder mehr aufeinanderfolgenden Dosen gentechnisch veränderter B-Zellen an ein Subjekt umfasst, wobei
(i) die Verabreichung zwei, drei oder mehr Dosen der gentechnisch veränderten B-Zellen in suboptimalen Einzeldosis-Konzentrationen umfasst, und/oder
(ii) die gentechnisch veränderten B-Zellen für das Subjekt autolog oder allogen sind, und/oder
(iii) wobei das Subjekt ein Mensch ist.

15. Die rekombinanten B-Zellen zur Verwendung nach Anspruch 13 oder 14, wobei die B-Zellen zur intravenösen, intraperitonealen, subkutanen, intrathekalen oder intramuskulären Injektion formuliert sind.

## Revendications

1. Une cellule B recombinante comprenant un gène de follistatine, dans laquelle la cellule B a été transduite ou transposée avec le gène de follistatine lié de manière fonctionnelle à un promoteur.

2. La cellule B selon la revendication 1, dans laquelle le gène de follistatine est un gène de follistatine humaine, dans laquelle
(i) le gène de follistatine est un variant de site d'épissage humain de la follistatine FST-344,
(ii) la cellule B est une cellule B humaine, et/ou
(iii) le gène de follistatine code pour la séquence d'acides aminés décrite dans l'une quelconque des séquences SEQ ID NO : 1 à 4.

3. La cellule B selon la revendication 1 ou 2, dans laquelle :
(i) la cellule B comprend le gène de follistatine parce qu'elle a été transduite avec le gène de follistatine en utilisant un système de transposon, y compris dans laquelle le système de transposon est un système de transposon Sleeping Beauty ou un système de transposon Piggybac ; ou
(ii) la cellule B exprime le gène de la follistatine en raison d'une transduction par un virus portant le gène de la follistatine, y compris dans le cas où la cellule B comprend le gène de la follistatine parce qu'elle a été transduite avec un rétrovirus, un lentivirus, un adénovirus ou un virus adéno-associé comprenant le gène de la follistatine ; ou
(iii) la cellule B est modifiée génétiquement pour contenir le gène de la follistatine à l'aide d'une approche d'intégration ciblée, y compris dans le cas où l'intégration ciblée utilise une ou plusieurs nucléases à doigt de zinc, des nucléases effectrices de type activateur de transcription (TALENs) et/ou des systèmes CRISPR/Cas, y compris, mais sans s'y limiter, les systèmes CRISPR/Cas9 ; ou
(iv) la cellule B est modifiée génétiquement pour contenir le gène de follistatine par introduction d'un acide nucléique codant la follistatine en utilisant un procédé choisi dans le groupe constitué de vecteurs rétroviraux, vecteurs lentiviraux, vecteurs de virus adéno-associé, vecteurs adénoviraux, tous autres vecteurs viraux à ARN ou ADN, ADN et/ou ARN non viral codant la follistatine introduit en utilisant des moyens chimiques ou physiques tels que la lipofection, la complexation polycationique, l'électroporation, et similaires.

4. La cellule B selon l'une quelconque des revendications précédentes, dans laquelle la protéine de follistatine est sécrétée par la cellule B recombinante.

5. La cellule B recombinante selon l'une quelconque des revendications 1 à 4, dans laquelle la cellule B est
(i) CD20-, CD38- et CD138-,
(ii) CD20- et CD38+,
(iii) CD20-, CD38+ et CD138+, ou
(iv) CD20-, CD38+ et CD138-.

6. La cellule B recombinante selon l'une quelconque des revendications 1 à 5, dans laquelle la cellule B recombinante est dérivée de
(i) une cellule B obtenue à partir d'un sujet,
(ii) une cellule B dérivée d'une cellule obtenue à partir d'un sujet,
(iii) un progéniteur de cellule B obtenu à partir d'un sujet, ou
(iv) une cellule obtenue à partir du sujet qui a été dédifférenciée en cellule B ou en progéniteur de cellule B.

7. Un procédé in vitro de production de cellules B recombinantes selon l'une quelconque des revendications 1 à 6, dans lequel des cellules immunitaires, telles que des cellules CD19 positives, ont été collectées et isolées à partir du sang d'un sujet ; et modifiées génétiquement par
(i) la transposition ou la transduction des cellules avec de l'ADN codant la follistatine, par électroporation, par exemple en utilisant le système de transposon Sleeping Beauty ;
(ii) l'expansion ex vivo des cellules sélectionnées ; et
(iii) la différenciation ex vivo des cellules expansées en plasmocytes et/ou plasmablastes, dans lequel les cellules différenciées sont CD38(+) et CD20(-).

8. Le procédé selon la revendication 7, dans lequel les cellules B recombinantes sont
(i) modifiées génétiquement au 2e ou au 3e jour de culture,
(ii) récoltées en vue de leur administration à un sujet entre le 1er et le 12e jour de culture in vitro et au 4e, 5e, 6e, 7e ou 8e jour de culture après modification génétique, et/ou
(iii) récoltées à un moment de la culture où il est déterminé qu'elles ne produisent pas de niveaux significatifs de cytokines inflammatoires, et/ou
(iv) cultivées dans un système de culture qui comprend chacun de IL-2, IL-4, IL-10, IL-15, IL-21 et un ligand CD40 multimérisé, un ligand CD40 marqué HIS qui est multimérisé en utilisant un anticorps anti-His, pendant toute la période de culture avant et après modification génétique.

9. Le procédé selon la revendication 7 ou 8, comprenant en outre l'expansion des cellules B génétiquement modifiées avant leur administration au sujet, dans lequel :
(i) la population finale de cellules B génétiquement modifiées expansées présente un degré élevé de polyclonalité, et/ou
(ii) tout clone de cellule B particulier dans la population finale de cellules B génétiquement modifiées expansées comprend moins de 0,05 % ou moins de 0,2 % de la population totale de cellules B.

10. Le procédé selon l'une quelconque des revendications 7 à 9, dans lequel les cellules B génétiquement modifiées comprennent un polynucléotide codant pour un marqueur de sélection, ledit marqueur de sélection étant un gène DHFR humain présentant une résistance accrue au méthotrexate, contenant des mutations de substitution de la leucine en tyrosine au niveau de l'acide aminé 22 et de la phénylalanine en sérine au niveau de l'acide aminé 31.

11. Le procédé selon l'une quelconque des revendications 7 à 10, comprenant le traitement des cellules B génétiquement modifiées avec du méthotrexate avant la récolte pour administration, dans lequel le traitement au méthotrexate est compris entre 100 nM et 300 nM.

12. Cellules B recombinantes obtenues par le procédé selon l'une quelconque des revendications 7 à 11.

13. Les cellules B recombinantes selon l'une quelconque des revendications 1 à 5 ou 12 pour utilisation dans un procédé de traitement, de prévention ou d'amélioration d'un trouble musculaire, dans lequel le trouble musculaire est choisi dans le groupe constitué d'une dystrophie musculaire telle que la dystrophie musculaire de Duchenne, la dystrophie musculaire de Becker ou la dystrophie musculaire facio-scapulo-humérale, un trouble musculaire inflammatoire tel que la myosite à inclusions, une lésion ou un traumatisme musculaire, une non-utilisation musculaire telle qu'après un alitement prolongé ou une immobilisation d'un membre, la sarcopénie, l'amyotrophie spinale (SMA), la sclérose latérale amyotrophique (ALS), la maladie de Pompe, la maladie du central core, et une atrophie ou un affaiblissement musculaire tel qu'après vieillissement, cancer ou maladies chroniques.

14. Les cellules B recombinantes pour l'utilisation selon la revendication 13, dans lesquelles le procédé comprend l'administration de deux doses séquentielles ou plus de cellules B génétiquement modifiées à un sujet, dans lesquelles
(i) l'administration comprend deux, trois doses ou plus des cellules B génétiquement modifiées à des concentrations de dose unique sous-optimales, et/ou
(ii) les cellules B génétiquement modifiées sont autologues ou allogéniques pour le sujet, et/ou
(iii) dans lesquelles le sujet est humain.

15. Les cellules B recombinantes pour l'utilisation selon la revendication 13 ou 14, dans lesquelles les cellules B sont formulées pour une injection intraveineuse, intrapéritonéale, sous-cutanée, intrathécale ou intramusculaire.
